# EUROPEAN PATENT SPECIFICATION

(11) **EP 1 916 311 B1**
(45) Date of publication and mention of the grant of the patent: **27.06.2012**
(21) Application number: 07118804.9
(22) Date of filing: 18.10.2007
(51) Int. Cl.: C12Q 1/68

(54) **Rolling circle amplification of circular genomes**
Rolling-Circle-Vermehrung zirkulärer Genome
Amplification de génomes circulaires par rolling circle

(30) Priority: 24.10.2006 US 862678 P
(43) Date of publication of application: 30.04.2008
(73) Proprietor: Qiagen GmbH, 40724 Hilden (DE)
(72) Inventor: Korfhage, Christian, 40764 Langenfeld (DE); Löffert, Dirk, 40589 Düsseldorf (DE)
(74) Representative: Kilger, Christian

(56) References cited:
- WO-A-2004/058987
- US-B1- 6 323 009
- RECTOR A ET AL: "A sequence-independent strategy for detection and cloning of circular DNA virus genomes by using multiply primed rolling-circle amplification" JOURNAL OF VIROLOGY, THE AMERICAN SOCIETY FOR MICROBIOLOGY, US, vol. 78, May 2004 (2004-05), pages 4993-4998, XP002392144 ISSN: 0022-538X
- BOORE J.L. ; MACEY J.R. ; MEDINA M.: "Sequencing and comparing whole mitochondrial genomes ofanimals" ELSEVIER, [Online] 22 April 2005 (2005-04-22), pages 1-62, XP002464504 Retrieved from the Internet: URL:http://www.osti.gov/energycitations/pr oduct.biblio.jsp?query_id=1&page=0&osti_id =861135>
- GE HEALTHCARE LIFE SCIENCES: 'TempliPhi FAQ' INTERNET CITATION, [Online] Retrieved from the Internet: <URL:http://www.gelifesciences.com/aptrix/u pp01077.nsf/Content/sample_preparation~prod uct_selection_category~rolling_circle_ampli fication~sample_templiphi_faq> [retrieved on 2010-07-29]
- DEAN F B ET AL: "Rapid amplification of plasmid and phage DNA using Phi 29 DNA polymerase and multiply-primed rolling circle amplification", GENOME RESEARCH, COLD SPRING HARBOR LABORATORY PRESS, WOODBURY, NY, US, vol. 11, no. 6, 1 June 2001 (2001-06-01), pages 1095-1099, XP002531577, ISSN: 1088-9051, DOI: 10.1101/GR.180501
- R. Fujii: "One-step random mutagenesis by error-prone rolling circle amplification", Nucleic Acids Research, vol. 32, no. 19, 28 October 2004 (2004-10-28), pages e145-e145, XP55011107, ISSN: 0305-1048, DOI: 10.1093/nar/gnh147

## Description

The disclosed invention is generally in the field of nucleic acid amplification.

### BACKGROUND OF THE INVENTION

A number of methods have been developed for exponential amplification of nucleic acids. These include the polymerase chain reaction (PCR), ligase chain reaction (LCR), self-sustained sequence replication (3SR), nucleic acid sequence based amplification (NASBA), strand displacement amplification (SDA), and amplification with Qβ replicase (Birkenmeyer and Mushahwar, J. Virological Methods, 35:117-126 (1991); Landegren, Trends Genetics 9:199-202 (1993)).

Fundamental to most genetic analysis is availability of genomic DNA of adequate quality and quantity. Techniques for whole genome amplification (WGA) by have been developed. Whole genome PCR, a variant of PCR amplification, involves the use of random or partially random primers to amplify the entire genome of an organism in the same PCR reaction. Whole genome amplification via Multiple Displacement Amplification (MDA) is an isothermic reaction involving strand displacement amplification that can provide high quality amplification of genomes of high complexity. This is described in U.S. Patent No. 6,124,120 to Lizardi. Amplification proceeds by replication initiating at each primer and continuing so that the growing strands encounter and displace adjacent replicated strands.

Rolling Circle Amplification (RCA) driven by DNA polymerase has been used to replicate circular oligonucleotide probes with either linear or geometric kinetics under isothermal conditions (Lizardi et al., Nature Genet. 19: 225-232 (1998); U.S. Patent Nos. 5, 854,033 and 6,143,495; PCT Application No. WO 97/19193). If a single primer is used, RCA generates in a few minutes a linear chain of hundreds or thousands of tandemly-linked DNA copies of a target that is covalently linked to that target. Generation of a linear amplification product permits both spatial resolution and accurate quantitation of a target. DNA generated by RCA can be labeled with fluorescent oligonucleotide tags that hybridize at multiple sites in the tandem DNA sequences. RCA can be used with fluorophore combinations designed for multiparametric color coding (PCT Application No. WO 97/19193), thereby markedly increasing the number of targets that can be analyzed simultaneously. RCA technologies can be used in solution, *in situ* and in microarrays. In solid phase formats, detection and quantitation can be achieved at the level of single molecules (Lizardi et al., 1998). Ligation-mediated Rolling Circle Amplification (LM-RCA) involves circularization of a probe molecule hybridized to a target sequence and subsequent rolling circle amplification of the circular probe (U.S. Patent Nos. 5, 854,033 and 6,143,495; PCT Application No. WO 97/19193). Very high yields of amplified products can be obtained with exponential rolling circle amplification (U.S. Patent Nos. 5, 854,033 and 6,143,495; PCT Application No. WO 97/19193) and multiply-primed rolling circle amplification (Dean et al., Genome Research 11:1095-1099 (2001)).

The document Rector et al., Journal of Virology, 78, 4993-4998, (2004) discloses a method of amplifying viral DNA genomes by multiply primed Rolling-Circle Amplification employing random hexamers and Phi29 polymerase. The method is exemplified by amplification of HPV-16 from a cell line. The smallest fold amplification was 71-fold and the largest was 2800-fold and depended on the amount of dNTP added to the amplification mix.

US-B1-6 323 009 discloses a method of amplifying circular DNA targets by multiply-primed RCA employing random or specific primers and discloses specifically the use of multiple specific primers and further the amplification of viral circular genomes.

### BRIEF SUMMARY OF THE INVENTION

The invention pertains to a method for amplification of circular genome as defined in claim 1. The method is based on rolling circle amplification of the circular genomes which involves strand displacement replication by primers. The disclosed method allows differential amplification of circular genomes of interest. In genomic nucleic acid samples containing both a circular genome of interest and non-target nucleic acids, such as non-target genomes, the disclosed method can result in many-fold differential amplification of the circular genome of interest over non-target nucleic acids. It has been discovered that selection of a set of primers complementary to a circular genome of interest can result in much greater amplification of the circular genome of interest relative to non-target nucleic acids present. Such differential amplification of circular genomes is very useful for obtaining useful amounts of genomes of interest from a mixed nucleic acid sample. For example, mitochondrial genomes, which, absent complicated and time consuming purification, are in the presence of non-target nucleic acids (such as the host cell genome), can be differentially amplified relative to the host cell genome and other non-target nucleic acids using the disclosed methods and composition.

The method involves bringing into contact a set of primers, DNA polymerase, and a genomic nucleic acid sample, where the genomic nucleic acid sample comprises a circular genome, and incubating the genomic nucleic acid sample under conditions that promote replication of the circular genome in the genomic nucleic acid sample. Replication of the circular genome can proceed by rolling circle replication. The conditions that promote replication of the circular genome does not involve thermal cycling and/or can be substantially isothermic. In the disclosed methods, the circular genome is differentially replicated compared to the non-target nucleic acids present in the genomic nucleic acid sample. Thus, for example, non-target nucleic acids present in the genomic nucleic acid sample generally would not be substantially, significantly, or notably replicated. For example, the primers in the set of primers and the reaction conditions generally can be selected such that non-target nucleic acids in the genomic nucleic acid sample are not substantially, significantly, or notably replicated.

Replication of the circular genomes results in replicated strands. Such replication proceeds by rolling circle replication to produce tandem sequence DNA. The replicated strands are displaced from the nucleic acid molecules by strand displacement replication of another replicated strand. Such amplification can proceed by replication with a highly processive polymerase initiating at each primer and continuing until spontaneous termination. A useful feature of the method is that as a DNA polymerase extends a primer, the polymerase displaces the replication products (that is, DNA strands) that resulted from extension of other primers. The polymerase is continuously extending new primers and displacing the replication products of previous priming events. In this way, multiple overlapping copies of all of the nucleic acid molecules and sequences in the circular genome can be synthesized in a short time. The disclosed method has advantages over the polymerase chain reaction since it can be carried out under isothermal conditions. In the disclosed method amplification takes place not in cycles, but in a continuous, isothermal replication. This makes amplification less complicated and much more consistent in output. Strand displacement allows rapid generation of multiple copies of a nucleic acid sequence or sample in a single, continuous, isothermal reaction.

When the genomic nucleic acid sample comprises one or more non-target genomes (including non-target genomes that may be circular), the circular genome of interest can be differentially amplified relative to the non-target genomes. The genomic nucleic acid sample can comprises a plurality of genomes including the circular genome of interest and one or more non-target genomes. Thus, for example, non-target nucleic acids present in the genomic nucleic acid sample generally would not be substantially, significantly, or notably replicated and/or amplified. For example, the primers in the set of primers and the reaction conditions generally can be selected such that non-target nucleic acids in the genomic nucleic acid sample are not substantially, significantly, or notably replicated and/or amplified.

The differential amplification of the circular genome can be described in quantitative terms. For example, the circular genome can be amplified, for example, at least 10 fold, 50 fold, 100 fold, 200 fold, 500 fold, 1000 fold, 2000 fold, or 5000 fold compared to the non-target nucleic acids and/or non-target genomes. Such differential amplification generally can be assessed by measuring the relative amplification of selected sequences within the circular genome and within one or more non-target nucleic acids and/or non -target genomes. Such assessments are described elsewhere herein.

The circular genome to be amplified can be any circular genome of interest. Circular genomes include, for example, organelle genomes, mitochondrial genomes, chloroplast genomes, plastid genomes, bacterial plasmid genomes, viral genomes, bacterial genomes, microbial genomes, and/or pathogen genomes. The circular genome can be a naturally occurring genome, a genome that is not artificially modified, and/or a genome that is not an artificial nucleic acid. The circular genome can be double-stranded, single-stranded, or partially double-stranded. Circular genomes occur in a variety of sizes and the disclosed methods can be used to amplify circular genomes of any size. For example, small viral genomes are typically between 5 and 40 kb, but some viral genomes are larger. Circular microbial genomes are known up to 1500 kb. Accordingly, the circular genome to be amplified can have, for example, a length of from about 3000 to about 300000 nucleotides, about 4000 to about 260000 nucleotides, about 5000 to about 150000 nucleotides, or about 5500 to about 40000 nucleotides.

The non-target genomes can be any genome that may be in a genomic nucleic acid sample. For example, non-target genomes can be, for example, bacterial genomes, viral genomes, microbial genomes, pathogen genomes, eukaryotic genomes, plant genomes, animal genomes, vertebrate genomes, fish genomes, avian genomes, mammalian genomes, rodent genomes, murine genomes, human genomes, host genomes, and/or non-target circular genomes. Circular genomes can be in the presence of non-target genomes. For example, organelle genomes are commonly in the presence of the cell genome of the cell in which the organelle resides. Pathogen genomes are commonly in the presence of host cell genomes.

It has been discovered that design and selection of primers for a set of primers allows differential amplification of circular genomes as described herein. The primers can each comprise or have a specific nucleotide sequence. All or a part of the specific nucleotide sequence can be complementary to a sequence in the circular genome. The primers can also include portions and/or sequences that are not complementary to the circular genome. The primers can specifically hybridize to a nucleotide sequence in the circular genome. For example, the primers can specifically hybridize to a nucleotide sequence in the circular genome under the conditions that promote replication of the circular genome. Generally each primer in a set of primers can hybridize to a different sequence and/or at a different location in the circular genome.

The primers can each have a nucleotide sequence complementary to a nucleotide sequence in the circular genome such that the primers hybridize at particular intervals on the circular genome. For example, the distance between consecutive primers hybridized to the same strand of the circular genome can average, for example, from about 200 to about 20000 nucleotides, about 200 to about 6000 nucleotides, about 300 to about 5000 nucleotides, or about 400 to about 4000 nucleotides. A minimum separation within such averages can also be specified. The distance between consecutive primers hybridized to the same strand of the circular genome can also be, for example, from about 200 to about 20000 nucleotides, about 200 to about 6000 nucleotides, about 300 to about 5000 nucleotides, or about 400 to about 4000 nucleotides.

When the circular genome is double-stranded or partially double-stranded, one or more of the primers can have a nucleotide sequence complementary to one of the strands of the circular genome and one or more of the primers can have a nucleotide sequence complementary to the other strand of the circular genome, all of the primers can have a nucleotide sequence complementary to one of the strands of the circular genome, or all of the primers can have a nucleotide sequence complementary to the other strand of the circular genome. Generally amplification will be more efficient if some of the primers are complementary to one strand and other primers are complementary to the other strand. Rolling circle replication of either or each strand of the circular genome will result in the formation of tandem sequence DNA complementary to that strand.

When the circular genome is single-stranded, the primers can have a nucleotide sequence complementary to the circular genome. Rolling circle replication of the single-stranded circular genome will result in the formation of tandem sequence DNA complementary to the circular genome. In some forms of the method, one or more of the primers can also have a nucleotide sequence that matches a sequence of the circular genome. In this case, the one or more of the primers that have a nucleotide sequence that matches a sequence of the circular genome can prime strand displacement replication of the tandem sequence DNA produced by rolling circle replication of the single-stranded circular genome by the primers that have a nucleotide sequence complementary to the circular genome. Replication of the tandem sequence DNA results in formation of secondary tandem sequence DNA.

When the circular genome is partially double-stranded (for example, where one strand is a continuous, closed circular strand and the other strand is discontinuous and non-circular), the primers can have a nucleotide sequence complementary to the circular strand of the circular genome. Rolling circle replication of the circular strand will result in the formation of tandem sequence DNA complementary to the circular strand. In some forms of the method, one or more of the primers can also have a nucleotide sequence that matches a sequence of the circular strand of the circular genome and/or is complementary to the non-circular strand of the circular genome. In this case, the one or more of the primers that have a nucleotide sequence that matches a sequence of the circular genome and/or is complementary to the non-circular strand of the circular genome can prime strand displacement replication of the tandem sequence DNA produced by rolling circle replication of the circular strand of the circular genome by the primers that have a nucleotide sequence complementary to the circular strand of the circular genome. Replication of the tandem sequence DNA results in formation of secondary tandem sequence DNA.

The primers can be composed in various ways. For example, the primers can each separately comprise deoxyribonucleotides, ribonucloetides, modified nucleotides, nucleotide analogs, labelled nucleotides, oligomer analogs, or a combination. In some forms of the disclosed methods and compositions, the primers can each contain at least one modified nucleotide such that the primers are nuclease resistant.

The primers can have any length that allows differential amplification of the circular genome of interest. The primers can have a complementary portion that can have any length that allows differential amplification of the circular genome of interest. For example, the primers can each separately have a length of, and/or can have a complementary portion having a length of, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, or 30 nucleotides. As another example, the primers can each separately have a length of, and/or can have a complementary portion having a length of, less than 6 nucleotides, less than 7 nucleotides, less than 8 nucleotides, less than 9 nucleotides, less than 10 nucleotides, less than 11 nucleotides, less than 12 nucleotides, less than 13 nucleotides, less than 14 nucleotides, less than 15 nucleotides, less than 16 nucleotides, less than 17 nucleotides, less than 18 nucleotides, less than 19 nucleotides, less than 20 nucleotides, less than 21 nucleotides, less than 22 nucleotides, less than 23 nucleotides, less than 24 nucleotides, less than 25 nucleotides, less than 26 nucleotides, less than 27 nucleotides, less than 28 nucleotides, less than 29 nucleotides, less than 30 nucleotides, or less than 31 nucleotides.

The set of primers can include any number of primers that allows differential amplification of the circular genome of interest. Generally, the number of primers can vary based on the size of the circular genome. The number of primers can also vary based on whether the circular genome is single-stranded or double-stranded. The number of primers can also vary based on the desired spacing between the primers when hybridized to the circular genome. As an example, the set of primers can comprise 2 primers, 3 primers, 4 primers, 5 primers, 6 primers, 7 primers, 8 primers, 9 primers, 10 primers, 11 primers, 12 primers, 13 primers, 14 primers, 15 primers, 16 primers, 17 primers, 18 primers, 19 primers, 20 primers, 21 primers, 22 primers, 23 primers, 24 primers, 25 primers, 26 primers, 27 primers, 28 primers, 29 primers, 30 primers, 31 primers, 32 primers, 33 primers, 34 primers, 35 primers, 36 primers, 37 primers, 38 primers, 39 primers, 40 primers, 41 primers, 42 primers, 43 primers, 44 primers, 45 primers, 46 primers, 47 primers, 48 primers, 49 primers, 50 primers, 51 primers, 52 primers, 53 primers, 54 primers, 55 primers, 56 primers, 57 primers, 58 primers, 59 primers, 60 primers, 61 primers, 62 primers, 63 primers, 75 primers, 100 primers, 150 primers, 200 primers, 300 primers, or 400 primers.

Any material or sample containing or suspected of containing a circular genome of interest can be used as the genomic nucleic acid sample. For example, the genomic nucleic acid sample can be a blood sample, a urine sample, a semen sample, a lymphatic fluid sample, a cerebrospinal fluid sample, amniotic fluid sample, a biopsy sample, a needle aspiration biopsy sample, a cancer sample, a tumor sample, a tissue sample, a cell sample, a cell lysate sample, a crude cell lysate sample, a forensic sample, an archeological sample, an infection sample, a nosocomial infection sample, an environmental sample, or a combination thereof.

Also disclosed is a method of identifying a set of primers for differential amplification of a circular genome. Such a method can generally involve selecting test primers for a test set of primers, bringing into contact the test set of primers, DNA polymerase, and a genomic nucleic acid sample, incubating the genomic nucleic acid sample under conditions that promote replication of the circular genome in the genomic nucleic acid sample, and determining the relative amplification of the circular genome and non-target nucleic acids. The test set of primers are identified if the circular genome is amplified at least 10 fold compared to the non-target nucleic acids. Generally, each primer can specifically hybridize to a nucleotide sequence in a circular genome of interest such that the distance between consecutive primers hybridized to the same strand of the circular genome averages from about 200 to about 20000 nucleotides, about 200 to about 6000 nucleotides. The test genomic nucleic acid sample comprises the circular genome and non-target nucleic acids. Replication of the circular genome proceeds by rolling circle replication. Generally, the conditions that promote replication of the circular genome do not involve thermal cycling and/or can be substantially isothermic.

Following amplification, the amplified sequences can be used for any purpose, such as uses known and established for amplified sequences. For example, amplified sequences can be detected using any of conventional detection systems for nucleic acids such as detection of fluorescent labels, enzyme-linked detection systems, antibody-mediated label detection, and detection of radioactive labels. A preferred form of labeling involves labeling of the replicated strands (that is, the strands produced in multiple displacement amplification) using terminal deoxynucleotidyl transferase. The replicated strands can be labeled by, for example, the addition of modified nucleotides, such as biotinylated nucleotides, fluorescent nucleotides, 5 methyl dCTP, BrdUTP, or 5-(3-aminoallyl)-2'-deoxyuridine 5'-triphosphates, to the 3' ends of the replicated strands. Amplification of forensic material for RFLP-based testing is one useful application for the disclosed method.

### BRIEF DESCRIPTION OF THE DRAWINGS

Figure 1 is a graph of the yield of amplification of mitochondrial DNA using either a set of primer each 10 nucleotides long or a set of primers each 14 nucleotides long.
Figure 2 is a graph of Ct for a mitochondrial locus and a nuclear locus obtained in real-time PCR of DNA amplified according to the disclosed method using a set of primer each 10 nucleotides long, DNA amplified according to the disclosed method using a set of primer each 14 nucleotides long, and unamplified source DNA.

### DETAILED DESCRIPTION OF THE INVENTION

The disclosed method makes use of certain materials and procedures which allow amplification of target nucleic acid sequences and whole genomes or other highly complex nucleic acid samples. These materials and procedures are described in detail below.

### Materials

Disclosed are materials, compositions, and components that can be used for, can be used in conjunction with, can be used in preparation for, or are products of the disclosed method and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a rolling circle replication primer is disclosed and discussed and a number of modifications that can be made to a number of molecules including the rolling circle replication primer are discussed, each and every combination and permutation of the rolling circle replication primer and the modifications that are possible are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, is this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### A. Circular Genome

The circular genome to be amplified can be any circular genome of interest, such as naturally occurring circular nucleic acids. Circular genomes include, for example, organelle genomes, mitochondrial genomes, chloroplast genomes, plastid genomes, bacterial plasmid genomes, viral genomes, bacterial genomes, microbial genomes, and/or pathogen genomes. The circular genome can be double-stranded, single-stranded, or partially double-stranded.

The circular genome can be a naturally occurring genome, a genome that is not artificially modified, and/or a genome that is not an artificial nucleic acid. As used herein, a naturally occurring genome is a genome that is substantially derived from natural processes and occurs in nature. A naturally occurring genome can include genetic modifications that were introduced unnaturally (such as via recombinant or transgenic DNA techniques) so long as the genome involved derived from natural processes and occurs in nature. Examples of naturally occurring genomes include organelle genomes, viral genomes, bacterial genomes, microbial genomes, and pathogen genomes. Examples of non-naturally occurring genomes include vectors and plasmids created via recombinant DNA and yeast artificial chromosomes. As used herein, a genome that is not artificially modified is a genome that has not been purposely modified such as via recombinant or transgenic DNA techniques. As used herein, an artificial nucleic acid is a nucleic acid purposely created using recombinant and other artificial, non-natural techniques.

Circular genomes occur in a variety of sizes and the disclosed methods can be used to amplify circular genomes of any size. For example, small viral genomes are typically between 5 and 40 kb, but some viral genomes are larger. Circular microbial genomes are known up to 1500 kb. Accordingly, the circular genome to be amplified can have, for example, a length of from about 3000 to about 300000 nucleotides, about 3000 to about 200000 nucleotides, about 3000 to about 150000 nucleotides, about 3000 to about 100000 nucleotides, about 3000 to about 75000 nucleotides, about 3000 to about 50000 nucleotides, about 3000 to about 40000 nucleotides, about 3000 to about 30000 nucleotides, about 3000 to about 25000 nucleotides, about 3000 to about 20000 nucleotides, about 3000 to about 18000 nucleotides, about 3000 to about 15000 nucleotides, about 3000 to about 12000 nucleotides, about 3000 to about 10000 nucleotides, about 3000 to about 8000 nucleotides, about 3000 to about 7000 nucleotides, about 3000 to about 6000 nucleotides, about 3000 to about 5000 nucleotides, about 3000 to about 4000 nucleotides, about 4000 to about 300000 nucleotides, about 5000 to about 300000 nucleotides, about 6000 to about 300000 nucleotides, about 7000 to about 300000 nucleotides, about 8000 to about 300000 nucleotides, about 10000 to about 300000 nucleotides, about 12000 to about 300000 nucleotides, about 15000 to about 300000 nucleotides, about 18000 to about 300000 nucleotides, about 20000 to about 300000 nucleotides, about 25000 to about 300000 nucleotides, about 30000 to about 300000 nucleotides, about 40000 to about 300000 nucleotides, about 50000 to about 300000 nucleotides, about 75000 to about 300000 nucleotides, about 100000 to about 300000 nucleotides, about 150000 to about 300000 nucleotides, about 200000 to about 300000 nucleotides, about 4000 to about 260000 nucleotides, about 4000 to about 200000 nucleotides, about 4000 to about 150000 nucleotides, about 4000 to about 40000 nucleotides, about 5000 to about 260000 nucleotides, about 5000 to about 200000 nucleotides, about 5000 to about 150000 nucleotides, about 5000 to about 40000 nucleotides, about 5500 to about 260000 nucleotides, about 5500 to about 200000 nucleotides, about 5500 to about 150000 nucleotides, about 5500 to about 40000 nucleotides, about 6000 to about 260000 nucleotides, about 6000 to about 200000 nucleotides, about 6000 to about 150000 nucleotides, about 6000 to about 40000 nucleotides, about 10000 to about 260000 nucleotides, about 10000 to about 200000 nucleotides, about 10000 to about 150000 nucleotides, or about 10000 to about 40000 nucleotides.

A circular genome can be in any nucleic acid sample of interest. The source, identity, and preparation of many such nucleic acid samples are known. It is preferred that nucleic acid samples known or identified for use in amplification or detection methods be used for the method described herein. The nucleic acid sample can be, for example, a nucleic acid sample from one or more cells, tissue, or bodily fluids such as blood, urine, semen, lymphatic fluid, cerebrospinal fluid, or amniotic fluid, or other biological samples, such as tissue culture cells, buccal swabs, mouthwash, stool, tissues slices, and biopsy aspiration. Genomic nucleic acid samples can be derived from any source including, but not limited to, eukaryotes, plants, animals, vertebrates, fish, mammals, humans, non-humans, bacteria, microbes, viruses, biological sources, serum, plasma, blood, urine, semen, lymphatic fluid, cerebrospinal fluid, amniotic fluid, biopsies, needle aspiration biopsies, cancers, tumors, tissues, cells, cell lysates, crude cell lysates, tissue lysates, tissue culture cells, buccal swabs, mouthwash, stool, forensic sources, autopsies, infections, nosocomial infections, and/or other terrestrial or extra-terrestrial materials and sources.

The sample may also contain mixtures of material from one or more different sources. For example, circular genomes of an infecting bacterium or virus can be amplified in the presence of human nucleic acids when circular genomes from such infected cells or tissues are amplified using the disclosed methods. Types of useful target samples include eukaryotic samples, plant samples, animal samples, vertebrate samples, fish samples, mammalian samples, human samples, non-human samples, bacterial samples, microbial samples, viral samples, biological samples, serum samples, plasma samples, blood samples, urine samples, semen samples, lymphatic fluid samples, cerebrospinal fluid samples, amniotic fluid samples, biopsy samples, needle aspiration biopsy samples, cancer samples, tumor samples, tissue samples, cell samples, cell lysate samples, crude cell lysate samples, tissue lysate samples, tissue culture cell samples, buccal swab samples, mouthwash samples, stool samples, forensic samples, autopsy samples, infection samples, nosocomial infection samples, and/or other terrestrial or extra-terrestrial samples.

### B. Genomic Nucleic Acid Samples

Genomic nucleic acid molecules can be any nucleic acid from any source. In general, the disclosed method is performed using a sample that contains (or is suspected of containing) circular genomes to be amplified. Samples containing, or suspected of containing, circular genomes can also be referred to as genomic nucleic acid samples. Samples, such as genomic nucleic acid samples can comprise circular genomes. Cell and tissue samples are a fonn of genomic nucleic acid sample. Samples for use in the disclosed methods can also be samples that are to be tested for the presence of circular genomes (that is, samples that may or may not contain circular genomes). A genomic nucleic acid sample is a form of nucleic acid sample and a form of sample. Reference herein to a sample encompasses nucleic acid samples and genomic samples unless the context clearly indicates otherwise. Reference herein to a nucleic acid sample encompasses genomic nucleic acid samples unless the context clearly indicates otherwise.

A sample can comprise a circular genome, and the circular genome can comprise any fraction of the nucleic acids in the sample. The circular genome can comprise, for example, at least 1 copy, at least 10 copies, at least 100 copies, at least 1000 copies, at least more than 1000 copies, or at least 0.001 %, at least 0.01 % , at least 0.1%, at least 1%, or more of the nucleic acids in the sample.

The nucleic acids in a sample need not be pure to be amplified in the disclosed methods. Some forms of the disclosed methods are useful for amplifying impure nucleic acid samples, such as crude cell lysates. The nucleic acids in a sample or in a stabilized or neutralized sample can be, for example, less than 0.01% pure, less than 0.5% pure, less than 0.1 % pure, less than 0.2% pure, less than 0.4% pure, less than 0.6% pure, less than 0.8% pure, less than 1% pure, less than 2% pure, less than 3% pure, less than 4% pure, less than 5% pure, less than 6% pure, less than 8% pure, less than 10% pure, less than 15% pure, less than 20% pure, less than 25% pure, less than 30% pure, less than 40% pure, or less than 50% pure by weight excluding water.

A genomic nucleic acid sample can be any nucleic acid sample of interest. The source, identity, and preparation of many such nucleic acid samples are known. It is preferred that nucleic acid samples known or identified for use in amplification or detection methods be used for the method described herein. The nucleic acid sample can be, for example, a nucleic acid sample comprising or derived from one or more eukaryotes, plants, animals, vertebrates, fish, mammals, humans, non-humans, bacteria, microbes, viruses, biological sources, serum, plasma, blood, urine, semen, lymphatic fluid, cerebrospinal fluid, amniotic fluid, biopsies, needle aspiration biopsies, cancers, tumors, tissues, cells, cell lysates, crude cell lysates, tissue lysates, tissue culture cells, buccal swabs, mouthwash, stool, forensic sources, autopsies, infections, nosocomial infections, and/or other terrestrial or extra-terrestrial materials and sources. Types of useful nucleic acid samples include eukaryotic samples, plant samples, animal samples, vertebrate samples, fish samples, mammalian samples, human samples, non-human samples, bacterial samples, microbial samples, viral samples, biological samples, serum samples, plasma samples, blood samples, urine samples, semen samples, lymphatic fluid samples, cerebrospinal fluid samples, amniotic fluid samples, biopsy samples, needle aspiration biopsy samples, cancer samples, tumor samples, tissue samples, cell samples, cell lysate samples, crude cell lysate samples, tissue lysate samples, tissue culture cell samples, buccal swab samples, mouthwash samples, stool samples, forensic samples, autopsy samples, infection samples, nosocomial infection samples, and/or other terrestrial or extra-terrestrial samples.

It is unnecessary to have prior knowledge of whether or not a sample contains amplifiable circular genomes. Some forms of the disclosed methods can be employed to test whether or not a sample suspected of containing circular genomes actually does contain circular genomes. Production of amplified DNA from such samples using the disclosed method is evidence that the sample contained circular genomes.

The genomic nucleic acid sample can be from a single cell. The genomic nucleic acid sample can be, or can be derived from, for example, one or more whole genomes from the same or different organisms, tissues, cells or a combination; one or more partial genomes from the same or different organisms, tissues, cells or a combination; one or more whole chromosomes from the same or different organisms, tissues, cells or a combination; one or more partial chromosomes from the same or different organisms, tissues, cells or a combination; one or more chromosome fragments from the same or different organisms, tissues, cells or a combination; one or more artificial chromosomes; one or more yeast artificial chromosomes; one or more bacterial artificial chromosomes; one or more cosmids; or any combination of these.

Samples can be used and manipulated in the disclosed methods. For example, a sample can be exposed to alkaline conditions or brought into contact or mixed with a lysis solution or denaturing solution. Lysis can be performed, for example, by mild lysis solutions such as low salt solutions that result in hypotonic burst of cells or harsh lysis such as lysis solutions containing alkaline or chaotropic salts or organic sovents. Nucleic acid samples may be purified or may be crude lysates. Nucleic acid samples can be further manipulated by adding substances for different purposes such as e.g. stabilizing of biomoelcules, better accessibility of biomolecules, neutralizing inhibitory substances, degrading contaminating molecules, separation of biomolecules. Nucleic acid samples can be further manipulated by adding enzymes for different purpose e.g. eliminating proteins (proteases: Protease K, Trypsin, Chymotrypsin, Collagenase, or other enzymes deemed to be suitable by the artisan), elimanting nucleic acids (e.g. RNases, Exonucleases, see also U.S. Patent No. 6,242,220 Wahle et al.), polysaccharides (e.g. Amylase, Pectinases, Glycosylases, Hyaluronidase). As used herein, the term sample refers both to source samples, samples used in the disclosed methods in whole, and to portions of source samples used in the disclosed methods. Thus, for example, a portion of a source sample that is exposed to alkaline conditions is considered to be a sample itself. All or a portion of a sample can be exposed to alkaline conditions or brought into contact or mixed with a lysis solution or denaturing solution. Similarly, the pH of all or a portion of a sample exposed to alkaline conditions or brought into contact or mixed with a lysis solution or denaturing solution can be reduced, or all or a portion of a sample exposed to alkaline conditions or brought into contact with a lysis solution or denaturing solution can be brought into contact or mixed with a stabilization solution. All or a portion of the resulting stabilized or neutralized sample can be incubated under conditions that promote replication of nucleic acids. An amplification mixture can comprise all or a portion of a stabilized or neutralized sample. An amplification mixture is the reaction solution where nucleic acids are amplified.

### C. Primers

Primers for use in the disclosed amplification method are oligonucleotides having sequence complementary to a target sequence. In the disclosed method, the target sequence can be a sequence in a circular genome of interest or the complement of a sequence in a circular genome of interest. This sequence is referred to as the complementary portion of the primer. The complementary portion of a primer can be any length that supports specific and stable hybridization between the primer and the target sequence under the reaction conditions. Generally, for reactions at 37°C, this can be 10 to 35 nucleotides long or 10 to 24 nucleotides long. Generally, for reactions at 30°C, this can be, for example, 6 to 20 nucleotides long or 8 to 12 nucleotides long. The primers can be, for example, from 6 to 60 nucleotides long or 8 to 60 nucleotides long, and in particular, can be 6, 7, 8, 9, 10, 11, 12, 13, 14, 15, 16, 17, 18, 19, and/or 20 nucleotides long. The primers also can be, for example, at least 6 nucleotides long, at least 7 nucleotides long, at least 8 nucleotides long, at least 9 nucleotides long, at least 10 nucleotides long, at least 11 nucleotides long, and/or at least 12 nucleotides long.

It has been discovered that design and selection of primers for a set of primers allows differential amplification of circular genomes as described herein. The primers can each comprise or have a specific nucleotide sequence. All or a part of the specific nucleotide sequence can be complementary to a sequence in the circular genome. The primers can also include portions and/or sequences that are not complementary to the circular genome. The primers can specifically hybridize to a nucleotide sequence in the circular genome. For example, the primers can specifically hybridize to a nucleotide sequence in the circular genome under the conditions that promote replication of the circular genome. Generally each primer in a set of primers can hybridize to a different sequence and/or at a different location in the circular genome.

The primers used in an amplification reaction need not be all of the same length, although this is preferred. For example, the primers can each separately have a length of, and/or can have a complementary portion having a length of, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, or 30 nucleotides. As another example, the primers can each separately have a length of, and/or can have a complementary portion having a length of, less than 6 nucleotides, less than 7 nucleotides, less than 8 nucleotides, less than 9 nucleotides, less than 10 nucleotides, less than 11 nucleotides, less than 12 nucleotides, less than 13 nucleotides, less than 14 nucleotides, less than 15 nucleotides, less than 16 nucleotides, less than 17 nucleotides, less than 18 nucleotides, less than 19 nucleotides, less than 20 nucleotides, less than 21 nucleotides, less than 22 nucleotides, less than 23 nucleotides, less than 24 nucleotides, less than 25 nucleotides, less than 26 nucleotides, less than 27 nucleotides, less than 28 nucleotides, less than 29 nucleotides, less than 30 nucleotides, or less than 31 nucleotides.

It is preferred that, when hybridized to nucleic acid molecules in a nucleic acid sample, the primers hybridize at intervals that allow efficient amplification. This generally can be accomplished by using a number of primers in the amplification reaction such that the primers collectively will be complementary to sequence in the nucleic acid sample at desired intervals. Thus, for example, each primer can specifically hybridize to a nucleotide sequence in a circular genome of interest such that the distance between consecutive primers hybridized to the same strand of the circular genome averages from about 200 to about 20000 nucleotides, about 200 to about 6000 nucleotides.

The primers can specifically hybridize to a nucleotide sequence in a circular genome of interest such that the distance between consecutive primers hybridized to the same strand of the circular genome averages 10,000 nucleotides or less, 9,500 nucleotides or less, 9,000 nucleotides or less, 8,500 nucleotides or less, 8,000 nucleotides or less, 7,500 nucleotides or less, 7,000 nucleotides or less, 6,500 nucleotides or less, 6,000 nucleotides or less, 5,500 nucleotides or less, 5,000 nucleotides or less, 4,500 nucleotides or less, 4,000 nucleotides or less, 3,500 nucleotides or less, 3,000 nucleotides or less, 2,500 nucleotides or less, 2,000 nucleotides or less, 1,500 nucleotides or less, 1,000 nucleotides or less, 900 nucleotides or less, 800 nucleotides or less, 700 nucleotides or less, 600 nucleotides or less, 500 nucleotides or less, 400 nucleotides or less, 300 nucleotides or less, 200 nucleotides or less, 100 nucleotides or less, or 50 nucleotides or less. A minimum separation within such averages can also be specified.

The optimal interval or separation distance between primer complementary sequences (and thus, the optimum number of primers) will not be the same for all DNA polymerases, because this parameter is dependent on the net polymerization rate. To obtain an optimal yield in the disclosed method, the number of primers and their composition can be adjusted to suit the polymerase being used. Use of fewer primers is preferred when using a polymerase with a rapid polymerization rate. Use of more primers is preferred when using a polymerase with a slower polymerization rate. Also disclosed is a method of identifying a set of primers for differential amplification of a circular genome. Such a method can generally involve selecting test primers for a test set of primers, bringing into contact the test set of primers, DNA polymerase, and a genomic nucleic acid sample, incubating the genomic nucleic acid sample under conditions that promote replication of the circular genome in the genomic nucleic acid sample, and determining the relative amplification of the circular genome and non-target nucleic acids. The test set of primers are identified if the circular genome is amplified at least 10 fold compared to the non-target nucleic acids.

The following assay can also be used to determine optimal spacing with any circular genome of interest. The assay uses some combination of two, three, four, five, six, seven, eight, nine, ten, eleven, twelve, thirteen, fourteen, fifteen, sixteen, seventeen, eighteen, nineteen, and twenty or more primers. For a given circular genome of interest, each new primer reduces the average distance between complementary sequences in the circular genome. The number of primers can be varied systematically between a range of primer numbers (the average distance between priming sites varies with the number of primers used). A series of reactions can be performed in which the same nucleic acid sample is amplified using the different numbers of primers. The number of primers that produces the highest experimental yield of DNA is the optimal primer number for the specific DNA polymerase, or DNA polymerase plus accessory protein combination being used. This assay can also be used to determine optimal spacing with any polymerase.

DNA replication initiated at the sites in nucleic acid molecules where the primers hybridize will extend to and displace strands being replicated from primers hybridized at adjacent sites. Displacement of an adjacent strand makes it available for hybridization to another primer and subsequent initiation of another round of replication. This process is referred to herein as strand displacement replication.

When the circular genome is double-stranded or partially double-stranded, one or more of the primers can have a nucleotide sequence complementary to one of the strands of the circular genome and one or more of the primers can have a nucleotide sequence complementary to the other strand of the circular genome, all of the primers can have a nucleotide sequence complementary to one of the strands of the circular genome, or all of the primers can have a nucleotide sequence complementary to the other strand of the circular genome. Generally, amplification will be more efficient if some of the primers are complementary to one strand and other primers are complementary to the other strand. Rolling circle replication of either or each strand of the circular genome will result in the formation of tandem sequence DNA complementary to that strand.

When the circular genome is single-stranded, the primers can have a nucleotide sequence complementary to the circular genome. Rolling circle replication of the single-stranded circular genome will result in the formation of tandem sequence DNA complementary to the circular genome. In some forms of the method, one or more of the primers can also have a nucleotide sequence that matches a sequence of the circular genome. In this case, the one or more of the primers that have a nucleotide sequence that matches a sequence of the circular genome can prime strand displacement replication of the tandem sequence DNA produced by rolling circle replication of the single-stranded circular genome by the primers that have a nucleotide sequence complementary to the circular genome. Replication of the tandem sequence DNA results in formation of secondary tandem sequence DNA.

When the circular genome is partially double-stranded (for example, where one strand is a continuous, closed circular strand and the other strand is discontinuous and non-circular), the primers can have a nucleotide sequence complementary to the circular strand of the circular genome. Rolling circle replication of the circular strand will result in the formation of tandem sequence DNA complementary to the circular strand. In some forms of the method, one or more of the primers can also have a nucleotide sequence that matches a sequence of the circular strand of the circular genome and/or is complementary to the non-circular strand of the circular genome. In this case, the one or more of the primers that have a nucleotide sequence that matches a sequence of the circular genome and/or is complementary to the non-circular strand of the circular genome can prime strand displacement replication of the tandem sequence DNA produced by rolling circle replication of the circular strand of the circular genome by the primers that have a nucleotide sequence complementary to the circular strand of the circular genome. Replication of the tandem sequence DNA results in formation of secondary tandem sequence DNA. Primers that have a nucleotide sequence that matches a sequence of the circular genome can be referred to as secondary DNA strand displacement primers.

The set of primers can include any number of primers that allows differential amplification of the circular genome of interest. Generally, the number of primers can vary based on the size of the circular genome. The number of primers can also vary based on whether the circular genome is single-stranded or double-stranded. The number of primers can also vary based on the desired spacing between the primers when hybridized to the circular genome. For example, the set of primers can comprise 2 primers, 3 primers, 4 primers, 5 primers, 6 primers, 7 primers, 8 primers, 9 primers, 10 primers, 11 primers, 12 primers, 13 primers, 14 primers, 15 primers, 16 primers, 17 primers, 18 primers, 19 primers, 20 primers, 21 primers, 22 primers, 23 primers, 24 primers, 25 primers, 26 primers, 27 primers, 28 primers, 29 primers, 30 primers, 31 primers, 32 primers, 33 primers, 34 primers, 35 primers, 36 primers, 37 primers, 38 primers, 39 primers, 40 primers, 41 primers, 42 primers, 43 primers, 44 primers, 45 primers, 46 primers, 47 primers, 48 primers, 49 primers, 50 primers, 51 primers, 52 primers, 53 primers, 54 primers, 55 primers, 56 primers, 57 primers, 58 primers, 59 primers, 60 primers, 61 primers, 62 primers, 63 primers, 75 primers, 100 primers, 150 primers, 200 primers, 300 primers, or 400 primers. There is no fundamental upper limit to the number of primers that can be used. Generally, the number of primers used can be chosen based on the size of the circular genome to be amplified and the average spacing between primers. When multiple primers are used, the primers should each have a different specific nucleotide sequence.

The primers used in the disclosed method can be selected and/or designed to have certain desirable features and functional characteristics. The goal of primer selection and primer design can be obtaining better amplification results. For example, particular primers can be selected that result in the highest amplification yield (that is, the highest amount of increase in the amount of nucleic acid), and/or the differential amplification of the circular genome compared to non-target sequences. This can be determined by testing particular primers in amplification reactions using a nucleic acid sample of interest. Different primers may produce optimal results for different nucleic acid samples. However, the primer number and primer composition principles described herein will generally produce good amplification results with most circular genomes.

The primers can also have other characteristics that can, for example, increase amplification yield and reduce production of artifacts or artifactual amplification. For example, generation of primer dimer artifacts can be reduced by designing primers to avoid 3' end sequences that are complementary, either between primers or within the same primer. Such sequences to be avoided can be referred to as inter-complementary 3' ends. A useful measure of a primer's ability to avoid artifactual amplification is the lack or relative insignificance of amplification (that is, nucleic acid produced) when the primer is used in an amplification reaction without a nucleic acid sample.

The primers can be composed in various ways. For example, the primers can each separately comprise deoxyribonucleotides, ribonucloetides, modified nucleotides, nucleotide analogs, labelled nucleotides-oligomer-analogs-or-a combination. In some forms of the disclosed methods and compositions, the primers can each contain at least one modified nucleotide such that the primers are nuclease resistant.

The disclosed primers can have one or more modified nucleotides. Such primers are referred to herein as modified primers. Modified primers have several advantages. First, some forms of modified primers, such as RNA/ 2'-O-methyl RNA chimeric primers, have a higher melting temperature (Tm) than DNA primers. This increases the stability of primer hybridization and will increase strand invasion by the primers. This will lead to more efficient priming. Also, since the primers are made of RNA, they will be exonuclease resistant. Such primers, if tagged with minor groove binders at their 5' end, will also have better strand invasion of the template dsDNA.

Chimeric primers can also be used. Chimeric primers are primers having at least two types of nucleotides, such as both deoxyribonucleotides and ribonucleotides, ribonucleotides and modified nucleotides, or two different types of modified nucleotides. One form of chimeric primer is peptide nucleic acid/nucleic acid primers. For example, 5'-PNA-DNA-3' or 5'-PNA-RNA-3' primers may be used for more efficient strand invasion and polymerization invasion. The DNA and RNA portions of such primers can have random or degenerate sequences. Other forms of chimeric primers are, for example, 5'- (2'-O-Methyl) RNA-RNA-3' or 5'- (2'-O-Methyl) RNA-DNA-3'.

Many modified nucleotides (nucleotide analogs) are known and can be used in oligonucleotides. A nucleotide analog is a nucleotide which contains some type of modification to either the base, sugar, or phosphate moieties. Modifications to the base moiety would include natural and synthetic modifications of A, C, G, and T/U as well as different purine or pyrimidine bases, such as uracil-5-yl, hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Additional base modifications can be found for example in U.S. Pat. No. 3,687,808, Englisch et al., Angewandte Chemie, International Edition, 1991, 30, 613, and Sanghvi, Y. S., Chapter 15, Antisense Research and Applications, pages 289-302, Crooke, S. T. and Lebleu, B. ed., CRC Press, 1993. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex formation. Other modified bases are those that function as universal bases. Universal bases include 3-nitropyrrole and 5-nitroindole. Universal bases substitute for the normal bases but have no bias in base pairing. That is, universal bases can base pair with any other base. Primers composed, either in whole or in part, of nucleotides with universal bases are useful for reducing or eliminating amplification bias against repeated sequences in a target sample. This would be useful, for example, where a loss of sequence complexity in the amplified products is undesirable. Base modifications often can be combined with for example a sugar modification, such as 2'-O-methoxyethyl, to achieve unique properties such as increased duplex stability. There are numerous United States patents such as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, which detail and describe a range of base modifications. Each of these patents is herein incorporated by reference.

Nucleotide analogs can also include modifications of the sugar moiety. Modifications to the sugar moiety would include natural modifications of the ribose and deoxyribose as well as synthetic modifications. Sugar modifications include but are not limited to the following modifications at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C1 to C10, alkyl or C2 to C10 alkenyl and alkynyl. 2' sugar modifications also include but are not limited to -O[(CH₂)n O]m CH₃, -O(CH₂)n OCH₃, -O(CH₂)n NH₂, - O(CH₂)n CH₃, -O(CH₂)n -ONH₂, and -O(CH₂)nON[(CH₂)nCH₃)]₂, where n and m are from 1 to about 10.

Other modifications at-the 2' position include but are-not limited to: C1 to C10 lower alkyl, substituted lower alkyl, alkaryl, aralkyl, O-alkaryl or O-aralkyl, SH, SCH₃, OCN, Cl, Br, CN, CF3, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs may also have sugar mimetics such as cyclobutyl moieties in place of the pentofuranosyl sugar. There are numerous United States patents that teach the preparation of such modified sugar structures such as 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkages between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included. Numerous United States patents teach how to make and use nucleotides containing modified phosphates and include but are not limited to, 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5.625,050.

It is understood that nucleotide analogs need only contain a single modification, but may also contain multiple modifications within one of the moieties or between different moieties.

Nucleotide substitutes are molecules having similar functional properties to nucleotides, but which do not contain a phosphate moiety, such as peptide nucleic acid (PNA). Nucleotide substitutes are molecules that will recognize and hybridize to complementary nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts. Numerous United States patents disclose how to make and use these types of phosphate replacements and include but are not limited to 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

It is also understood in a nucleotide substitute that both the sugar and the phosphate moieties of the nucleotide can be replaced, by for example an amide type linkage (aminoethylglycine) (PNA). United States patents 5,539,082; 5,714,331; and 5,719,262 teach how to make and use PNA-molecules. (See also Nielsen et al., Science 254:1497-1500 (1991)).

Primers can be comprised of nucleotides and can be made up of different types of nucleotides or the same type of nucleotides. For example, one or more of the nucleotides in a primer can be ribonucleotides, 2'-O-methyl ribonucleotides, or a mixture of ribonucleotides and 2'-O-methyl ribonucleotides; about 10% to about 50% of the nucleotides can be ribonucleotides, 2'-O-methyl ribonucleotides, or a mixture of ribonucleotides and 2'-O-methyl ribonucleotides; about 50% or more of the nucleotides can be ribonucleotides, 2'-O-methyl ribonucleotides, or a mixture of ribonucleotides and 2'-O-methyl ribonucleotides; or all of the nucleotides are ribonucleotides, 2'-O-methyl ribonucleotides, or a mixture of ribonucleotides and 2'-O-methyl ribonucleotides. The nucleotides can be comprised of bases (that is, the base portion of the nucleotide) and can (and normally will) comprise different types of bases. For example, one or more of the bases can be universal bases, such as 3-nitropyrrole or 5-nitroindole; about 10% to about 50% of the bases can be universal bases; about 50% or more of the bases can be universal bases; or all of the bases can be universal bases.

Primers may, but need not, also contain additional sequence at the 5' end of the primer that is not complementary to the target sequence. This sequence is referred to as the non-complementary portion of the primer. The non-complementary portion of the primer, if present, serves to facilitate strand displacement during DNA replication. The non-complementary portion of the primer can also include a functional sequence such as a promoter for an RNA polymerase. The non-complementary portion of a primer may be any length, but is generally 1 to 100 nucleotides long, and preferably 4 to 8 nucleotides long. The use of a non-complementary portion is not preferred when random or partially random primers are used for whole genome amplification.

It is specifically contemplated that use of conditions that allow or are compatible with substantial, significant or notable mismatch hybridization of the primers to nucleic acid molecules being amplified can be excluded. As used herein, substantial mismatch hybridization of a primer refers to hybridization where 90% or more of the primer nucleotides are unpaired to nucleotides in the hybridization partner. Significant mismatch hybridization of a primer refers to hybridization where 50% or more of the primer nucleotides are unpaired to nucleotides in the hybridization partner. Notable mismatch hybridization of a primer refers to hybridization where 10% or more of the primer nucleotides are unpaired to nucleotides in the hybridization partner. Choosing conditions that avoid or that are not compatible with substantial or significant or notable mismatch hybridization of the primers emphasizes the use of specific or substantially specific hybridization of the primers in the disclosed method.

An important factor for conditions that do or do not allow, or that are or are not compatible with, a given level of mismatch hybridization is the temperature at which the amplification is carried out. Thus, for example, a temperature significantly below the melting temperature of a primer generally would allow a higher level of mismatch hybridization by that primer than a temperature closer to its melting temperature because hybrids involving only some of the nucleotides in the primer would be stable at the lower temperature. In this way, the reaction temperature (that is, the temperature at which the nucleic acid sample, primer and DNA polymerase are incubated for amplification) affects the level of mismatch hybridization and the intervals at which primers will hybridize to nucleotide sequences in the nucleic acid sample.

To make use of primer specificity in the disclosed method, the primers can be designed (or, conversely, the incubation temperature can be chosen) to reduce the level of mismatch hybridization. In general, this can involve use of lower incubation temperatures for shorter primers and higher incubation temperatures for longer primers. As deemed suitable and desirable, the primers can be designed for use at, and/or the amplification reaction can be incubated at 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, or 80°C. The primers can be designed for use at, and/or the amplification reaction can be incubated at less than 21°C, less than 22°C, less than 23°C, less than 24°C, less than 25°C, less than 26°C, less than 27°C, less than 28°C, less than 29°C, less than 30°C, less than 31°C, less than 32°C, less than 33°C, less than 34°C, less than 35°C, less than 36°C, less than 37°C, less than 38°C, less than 39°C, less than 40°C, less than 41 °C, less than 42°C, less than 43°C, less than 44°C, less than 45°C, less than 46°C, less than 47°C, less than 48°C, less than 49°C, less than 50°C, less than 51°C, less than 52°C, less than 53°C, less than 54°C, less than 55°C, less than 56°C, less than 57°C, less than 58°C, less than 59°C, less than 60°C, less than 61°C, less than 62°C, less than 63°C, less than 64°C, less than 65°C, less than 66°C, less than 67°C, less than 68°C, less than 69°C, less than 70°C, less than 71°C, less than 72°C, less than 73°C, less than 74°C, less than 75°C, less than 76°C, less than 77°C, less than 78°C, less than 79°C, or less than 80°C.

### 1. Detection Tags

The non-complementary portion of a primer can include sequences to be used to further manipulate or analyze amplified sequences. An example of such a sequence is a detection tag, which is a specific nucleotide sequence present in the non-complementary portion of a primer. Detection tags have sequences complementary to detection probes. Detection tags can be detected using their cognate detection probes. Detection tags become incorporated at the ends of amplified strands. The result is amplified DNA having detection tag sequences that are complementary to the complementary portion of detection probes. If present, there may be one, two, three, or more than three detection tags on a primer. It is preferred that a primer have one, two, three or four detection tags. Most preferably, a primer will have one detection tag. Generally, it is preferred that a primer have 10 detection tags or less. There is no fundamental limit to the number of detection tags that can be present on a primer except the size of the primer. When there are multiple detection tags, they may have the same sequence or they may have different sequences, with each different sequence complementary to a different detection probe. It is preferred that a primer contain detection tags that have the same sequence such that they are all complementary to a single detection probe. For some multiplex detection methods, it is preferable that primers contain up to six detection tags and that the detection tag portions have different sequences such that each of the detection tag portions is complementary to a different detection probe. A similar effect can be achieved by using a set of primers where each has a single different detection tag. The detection tags can each be any length that supports specific and stable hybridization between the detection tags and the detection probe. For this purpose, a length of 10 to 35 nucleotides is preferred, with a detection tag portion 15 to 20 nucleotides long being most preferred.

### 2. Address Tag

Another example of a sequence that can be included in the non-complementary portion of a primer is an address tag. An address tag has a sequence complementary to an address probe. Address tags become incorporated at the ends of amplified strands. The result is amplified DNA having address tag sequences that are complementary to the complementary portion of address probes. If present, there may be one, or more than one, address tag on a primer. It is preferred that a primer have one or two address tags. Most preferably, a primer will have one address tag. Generally, it is preferred that a primer have 10 address tags or less. There is no fundamental limit to the number of address tags that can be present on a primer except the size of the primer. When there are multiple address tags, they may have the same sequence or they may have different sequences, with each different sequence complementary to a different address probe. It is preferred that a primer contain address tags that have the same sequence such that they are all complementary to a single address probe. The address tag portion can be any length that supports specific and stable hybridization between the address tag and the address probe. For this purpose, a length between 10 and 35 nucleotides long is preferred, with an address tag portion 15 to 20 nucleotides long being most preferred.

### D. Fluorescent Change Probes and Primers

Fluorescent change probes and fluorescent change primers refer to all probes and primers that involve a change in fluorescence intensity or wavelength based on a change in the form or conformation of the probe or primer and nucleic acid to be detected, assayed or replicated. Examples of fluorescent change probes and primers include molecular beacons, Amplifluors, FRET probes, cleavable FRET probes, TaqMan probes, scorpion primers, fluorescent triplex oligos, fluorescent water-soluble conjugated polymers, PNA probes and QPNA probes.

Fluorescent change probes and primers can be classified according to their structure and/or function. Fluorescent change probes include hairpin quenched probes, cleavage quenched probes, cleavage activated probes, and fluorescent activated probes. Fluorescent change primers include stem quenched primers and hairpin quenched primers. The use of several types of fluorescent change probes and primers are reviewed in Schweitzer and Kingsmore, Curr. Opin. Biotech. 12:21-27 (2001). Hall et al., Proc. Natl. Acad. Sci. USA 97:8272-8277 (2000), describe the use of fluorescent change probes with Invader assays.

Hairpin quenched probes are probes that when not bound to a target sequence form a hairpin structure (and, typically, a loop) that brings a fluorescent label and a quenching moiety into proximity such that fluorescence from the label is quenched. When the probe binds to a target sequence, the stem is disrupted, the quenching moiety is no longer in proximity to the fluorescent label and fluorescence increases. Examples of hairpin quenched probes are molecular beacons, fluorescent triplex oligos, and QPNA probes.

Cleavage activated probes are probes where fluorescence is increased by cleavage of the probe. Cleavage activated probes can include a fluorescent label and a quenching moiety in proximity such that fluorescence from the label is quenched. When the probe is clipped or digested (typically by the 5'-3' exonuclease activity of a polymerase during amplification), the quenching moiety is no longer in proximity to the fluorescent label and fluorescence increases. TaqMan probes (Holland et al., Proc. Natl. Acad. Sci. USA 88:7276-7280 (1991)) are an example of cleavage activated probes.

Cleavage quenched probes are probes where fluorescence is decreased or altered by cleavage of the probe. Cleavage quenched probes can include an acceptor fluorescent label and a donor moiety such that, when the acceptor and donor are in proximity, fluorescence resonance energy transfer from the donor to the acceptor causes the acceptor to fluoresce. The probes are thus fluorescent, for example, when hybridized to a target sequence. When the probe is clipped or digested (typically by the 5'-3' exonuclease activity of a polymerase during amplification), the donor moiety is no longer in proximity to the acceptor fluorescent label and fluorescence from the acceptor decreases. If the donor moiety is itself a fluorescent label, it can release energy as fluorescence (typically at a different wavelength than the fluorescence of the acceptor) when not in proximity to an acceptor. The overall effect would then be a reduction of acceptor fluorescence and an increase in donor fluorescence. Donor fluorescence in the case of cleavage quenched probes is equivalent to fluorescence generated by cleavage activated probes with the acceptor being the quenching moiety and the donor being the fluorescent label. Cleavable FRET (fluorescence resonance energy transfer) probes are an example of cleavage quenched probes.

Fluorescent activated probes are probes or pairs of probes where fluorescence is increased or altered by hybridization of the probe to a target sequence. Fluorescent activated probes can include an acceptor fluorescent label and a donor moiety such that, when the acceptor and donor are in proximity (when the probes are hybridized to a target sequence), fluorescence resonance energy transfer from the donor to the acceptor causes the acceptor to fluoresce. Fluorescent activated probes are typically pairs of probes designed to hybridize to adjacent sequences such that the acceptor and donor are brought into proximity. Fluorescent activated probes can also be single probes containing both a donor and acceptor where, when the probe is not hybridized to a target sequence, the donor and acceptor are not in proximity but where the donor and acceptor are brought into proximity when the probe hybridized to a target sequence. This can be accomplished, for example, by placing the donor and acceptor on opposite ends a the probe and placing target complement sequences at each end of the probe where the target complement sequences are complementary to adjacent sequences in a target sequence. If the donor moiety of a fluorescent activated probe is itself a fluorescent label, it can release energy as fluorescence (typically at a different wavelength than the fluorescence of the acceptor) when not in proximity to an acceptor (that is, when the probes are not hybridized to the target sequence). When the probes hybridize to a target sequence, the overall effect would then be a reduction of donor fluorescence and an increase in acceptor fluorescence. FRET probes are an example of fluorescent activated probes.

Stem quenched primers are primers that when not hybridized to a complementary sequence form a stem structure (either an intramolecular stem structure or an intermolecular stem structure) that brings a fluorescent label and a quenching moiety into proximity such that fluorescence from the label is quenched. When the primer binds to a complementary sequence, the stem is disrupted, the quenching moiety is no longer in proximity to the fluorescent label and fluorescence increases. In the disclosed method, stem quenched primers are used as primers for nucleic acid synthesis and thus become incorporated into the synthesized or amplified nucleic acid. Examples of stem quenched primers are peptide nucleic acid quenched primers and hairpin quenched primers.

Peptide nucleic acid quenched primers are primers associated with a peptide nucleic acid quencher or a peptide nucleic acid fluor to form a stem structure. The primer contains a fluorescent label or a quenching moiety and is associated with either a peptide nucleic acid quencher or a peptide nucleic acid fluor, respectively. This puts the fluorescent label in proximity to the quenching moiety. When the primer is replicated, the peptide nucleic acid is displaced, thus allowing the fluorescent label to produce a fluorescent signal.

Hairpin quenched primers are primers that when not hybridized to a complementary sequence form a hairpin structure (and, typically, a loop) that brings a fluorescent label and a quenching moiety into proximity such that fluorescence from the label is quenched. When the primer binds to a complementary sequence, the stem is disrupted, the quenching moiety is no longer in proximity to the fluorescent label and fluorescence increases. Hairpin quenched primers are typically used as primers for nucleic acid synthesis and thus become incorporated into the synthesized or amplified nucleic acid. Examples of hairpin quenched primers are Amplifluor primers (Nazerenko et al., Nucleic Acids Res. 25:2516-2521 (1997)) and scorpion primers (Thelwell et al., Nucleic Acids Res. 28(19):3752-3761 (2000)).

Cleavage activated primers are similar to cleavage activated probes except that they are primers that are incorporated into replicated strands and are then subsequently cleaved. Little et al., Clin. Chem. 45:777-784 (1999), describe the use of cleavage activated primers.

### E. Detection Labels

To aid in detection and quantitation of nucleic acids amplified using the disclosed method, detection labels can be directly incorporated into amplified nucleic acids or can be coupled to detection molecules. As used herein, a detection label is any molecule that can be associated with amplified nucleic acid, directly or indirectly, and which results in a measurable, detectable signal, either directly or indirectly. Many such labels for incorporation into nucleic acids or coupling to nucleic acid probes are known to those of skill in the art. Examples of detection labels suitable for use in the disclosed method are radioactive isotopes, fluorescent molecules, phosphorescent molecules, enzymes, antibodies, and ligands.

Examples of suitable fluorescent labels include fluorescein isothiocyanate (FITC), 5,6-carboxymethyl fluorescein, Texas red, nitrobenz-2-oxa-1,3-diazol-4-yl (NBD), coumarin, dansyl chloride, rhodamine, amino-methyl coumarin (AMCA), Eosin, Erythrosin, BODIPY^{®}, Cascade Blue^{®}, Oregon Green^{®}, pyrene, lissamine, xanthenes, acridines, oxazines, phycoerythrin, macrocyclic chelates of lanthanide ions such-as quantum dye^{™}, fluorescent energy transfer dyes, such as thiazole orange-ethidium heterodimer, and the cyanine dyes Cy3, Cy3.5, Cy5, Cy5.5 and Cy7. Examples of other specific fluorescent labels include 3-Hydroxypyrene 5,8,10-Tri Sulfonic acid, 5-Hydroxy Tryptamine (5-HT), Acid Fuchsin, Alizarin Complexon, Alizarin Red, Allophycocyanin, Aminocoumarin, Anthroyl Stearate, Astrazon Brilliant Red 4G, Astrazon Orange R, Astrazon Red 6B, Astrazon Yellow 7 GLL, Atabrine, Auramine, Aurophosphine, Aurophosphine G, BAO 9 (Bisaminophenyloxadiazole), BCECF, Berberine Sulphate, Bisbenzamide, Blancophor FFG Solution, Blancophor SV, Bodipy F1, Brilliant Sulphoflavin FF, Calcien Blue, Calcium Green, Calcofluor RW Solution, Calcofluor White, Calcophor White ABT Solution, Calcophor White Standard Solution, Carbostyryl, Cascade Yellow, Catecholamine, Chinacrine, Coriphosphine O, Coumarin-Phalloidin, CY3.1 8, CY5.1 8, CY7, Dans (1-Dimethyl Amino Naphaline 5 Sulphonic Acid), Dansa (Diamino Naphtyl Sulphonic Acid), Dansyl NH-CH3, Diamino Phenyl Oxydiazole (DAO), Dimethylamino-5-Sulphonic acid, Dipyrrometheneboron Difluoride, Diphenyl Brilliant Flavine 7GFF, Dopamine, Erythrosin ITC, Euchrysin, FIF (Formaldehyde Induced Fluorescence), Flazo Orange, Fluo 3, Fluorescamine, Fura-2, Genacryl Brilliant Red B, Genacryl Brilliant Yellow 10GF, Genacryl Pink 3G, Genacryl Yellow 5GF, Gloxalic Acid, Granular Blue, Haematoporphyrin, Indo-1, Intrawhite Cf Liquid, Leucophor PAF, Leucophor SF, Leucophor WS, Lissamine Rhodamine B200 (RD200), Lucifer Yellow CH, Lucifer Yellow VS, Magdala Red, Marina Blue, Maxilon Brilliant Flavin 10 GFF, Maxilon Brilliant Flavin 8 GFF, MPS (Methyl Green Pyronine Stilbene), Mithramycin, NBD Amine, Nitrobenzoxadidole, Noradrenaline, Nuclear Fast Red, Nuclear Yellow, Nylosan Brilliant Flavin E8G, Oxadiazole, Pacific Blue, Pararosaniline (Feulgen), Phorwite AR Solution, Phorwite BKL, Phorwite Rev, Phorwite RPA, Phosphine 3R, Phthalocyanine, Phycoerythrin R, Polyazaindacene Pontochrome Blue Black, Porphyrin, Primuline, Procion Yellow, Pyronine, Pyronine B, Pyrozal Brilliant Flavin 7GF, Quinacrine Mustard, Rhodamine 123, Rhodamine 5 GLD, Rhodamine 6G, Rhodamine B, Rhodamine B 200, Rhodamine B Extra, Rhodamine BB, Rhodamine BG, Rhodamine WT, Serotonin, Sevron Brilliant Red 2B, Sevron Brilliant Red 4G, Sevron Brilliant Red B, Sevron Orange, Sevron Yellow L, SITS (Primuline), SITS (Stilbene Isothiosulphonic acid), Stilbene, Snarf 1, sulpho Rhodamine B Can C, Sulpho Rhodamine G Extra, Tetracycline, Thiazine Red R, Thioflavin S, Thioflavin TCN, Thioflavin 5, Thiolyte, Thiozol Orange, Tinopol CBS, True Blue, Ultralite, Uranine B, Uvitex SFC, Xylene Orange, and XRITC.

Preferred fluorescent labels are fluorescein (5-carboxyfluorescein-N-hydroxysuccinimide ester), rhodamine (5,6-tetramethyl rhodamine), and the cyanine dyes Cy3, Cy3.5, Cy5, Cy5.5 and Cy7. The absorption and emission maxima, respectively, for these fluors are: FITC (490 nm; 520 nm), Cy3 (554 nm; 568 nm), Cy3.5 (581 nm; 588 nm), Cy5 (652 nm: 672 nm), Cy5.5 (682 nm; 703 nm) and Cy7 (755 nm; 778 nm), thus allowing their simultaneous detection. Other examples of fluorescein dyes include 6-carboxyfluorescein (6-FAM), 2',4',1,4,-tetrachlorofluorescein (TET), 2',4',5',7',1,4-hexachlorofluorescein (HEX), 2',7'-dimethoxy-4', 5'-dichloro-6-carboxyrhodamine (JOE), 2'-chloro-5'-fluoro-7',8'-fused phenyl-1,4-dichloro-6-carboxyfluorescein (NED), and 2'-chloro-7'-phenyl-1,4-dichloro-6-carboxyfluorescein (VIC). Fluorescent labels can be obtained from a variety of commercial sources, including Amersham Pharmacia Biotech, Piscataway, NJ; Molecular Probes, Eugene, OR; and Research Organics, Cleveland, Ohio.

Additional labels of interest include those that provide for signal only when the probe with which they are associated is specifically bound to a target molecule, where such labels include: "molecular beacons" as described in Tyagi & Kramer, Nature Biotechnology (1996) 14:303 and EP 0 070 685 B1. Other labels of interest include those described in U.S. Pat. No. 5,563,037; WO 97/17471 and WO 97/17076.

Labeled nucleotides are a preferred form of detection label since they can be directly incorporated into the amplification products during synthesis. Examples of detection labels that can be incorporated into amplified nucleic acids include nucleotide analogs such as BrdUrd (5-bromodeoxyuridine, Hoy and Schimke, Mutation Research 290:217-230 (1993)), aminoallyldeoxyuridine (Henegariu et al., Nature Biotechnology 18:345-348 (2000)), 5-methylcytosine *(*Sano et al., Biochim. Biophys. Acta 951:157-165 (1988)), bromouridine (Wansick et al., J. Cell Biology 122:283-293 (1993)) and nucleotides modified with biotin (Langer et al., Proc. Natl. Acad. Sci. USA 78:6633 (1981)) or with suitable haptens such as digoxygenin (Kerkhof, Anal. Biochem. 205:359-364 (1992)). Suitable fluorescence-labeled nucleotides are Fluorescein-isothiocyanate-dUTP, Cyanine-3-dUTP and Cyanine-5-dUTP (Yu et al., Nucleic Acids Res., 22:3226-3232 (1994)). A preferred nucleotide analog detection label for DNA is BrdUrd (bromodeoxyuridine, BrdUrd, BrdU, BUdR, Sigma-Aldrich Co). Other preferred nucleotide analogs for incorporation of detection label into DNA are AA-dUTP (aminoallyl-deoxyuridine-triphosphate-Sigma-Aldrich Co.), and 5-methyl-dCTP (Roche Molecular Biochemicals). A preferred nucleotide analog for incorporation of detection label into RNA is biotin-16-UTP (biotin-16-uridine-5'-triphosphate, Roche Molecular Biochemicals). Fluorescein, Cy3, and Cy5 can be linked to dUTP for direct labelling. Cy3.5 and Cy7 are available as avidin or anti-digoxygenin conjugates for secondary detection of biotin- or digoxygenin-labeled probes.

Detection labels that are incorporated into amplified nucleic acid, such as biotin, can be subsequently detected using sensitive methods well-known in the art. For example, biotin can be detected using streptavidin-alkaline phosphatase conjugate (Tropix, Inc.), which is bound to the biotin and subsequently detected by chemiluminescence of suitable substrates (for example, chemiluminescent substrate CSPD: disodium, 3-(4-methoxyspiro-[1,2,-dioxetane-3-2'-(5'-chloro)tricyclo [3.3.1.1^{3,7}]decane]-4-yl) phenyl phosphate; Tropix, Inc.). Labels can also be enzymes, such as alkaline phosphatase, soybean peroxidase, horseradish peroxidase and polymerases, that can be detected, for example, with chemical signal amplification or by using a substrate to the enzyme which produces light (for example, a chemiluminescent 1,2-dioxetane substrate) or fluorescent signal.

Molecules that combine two or more of these detection labels are also considered detection labels. Any of the known detection labels can be used with the disclosed probes, tags, and method to label and detect nucleic acid amplified using the disclosed method. Methods for detecting and measuring signals generated by detection labels are also known to those of skill in the art. For example, radioactive isotopes can be detected by scintillation counting or direct visualization; fluorescent molecules can be detected with fluorescent spectrophotometers; phosphorescent molecules can be detected with a spectrophotometer or directly visualized with a camera; enzymes can be detected by detection or visualization of the product of a reaction catalyzed by the enzyme; antibodies can be detected by detecting a secondary detection label coupled to the antibody. As used herein, detection molecules are molecules which interact with amplified nucleic acid and to which one or more detection labels are coupled.

### F. Detection Probes

Detection probes are labeled oligonucleotides having sequence complementary to detection tags or another sequence on amplified nucleic acids. The complementary portion of a detection probe can be any length that supports specific and stable hybridization between the detection probe and its complementary sequence on the amplified DNA. For this purpose, a length of 10 to 35 nucleotides is preferred, with a complementary portion of a detection probe 16 to 20 nucleotides long being most preferred. Detection probes can contain any of the detection labels described above. Preferred labels are biotin and fluorescent molecules. A particularly preferred detection probe is a molecular beacon. Molecular beacons are detection probes labeled with fluorescent moieties where the fluorescent moieties fluoresce only when the detection probe is hybridized (Tyagi and Kramer, Nature Biotechnol. 14:303-309 (1995)). The use of such probes eliminates the need for removal of unhybridized probes prior to label detection because the unhybridized detection probes will not produce a signal. This is especially useful in multiplex assays.

### G. Address Probes

An address probe is an oligonucleotide having a sequence complementary to address tags on primers. The complementary portion of an address probe can be any length that supports specific and stable hybridization between the address probe and the address tag. For this purpose, a length of 10 to 35 nucleotides is preferred, with a complementary portion of an address probe 12 to 18 nucleotides long being most preferred. An address probe can contain a single complementary portion or multiple complementary portions. Preferably, address probes are coupled, either directly or via a spacer molecule, to a solid-state support. Such a combination of address probe and solid-state support are a preferred form of solid-state detector.

### H. Oligonucleotide Synthesis

Primers, detection probes, address probes, and any other oligonucleotides can be synthesized using established oligonucleotide synthesis methods. Methods to produce or synthesize oligonucleotides are well known in the art. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method. Solid phase chemical synthesis of DNA fragments is routinely performed using protected nucleoside cyanoethyl phosphoramidites (S. L. Beaucage et al. (1981) Tetrahedron Lett. 22:1859). In this approach, the 3'-hydroxyl group of an initial 5'-protected nucleoside is first covalently attached to the polymer support (R. C. Pless et al. (1975) Nucleic Acids Res. 2:773 (1975)). Synthesis of the oligonucleotide then proceeds by deprotection of the 5'-hydroxyl group of the attached nucleoside, followed by coupling of an incoming nucleoside-3'-phosphoramidite to the deprotected hydroxyl group (M. D. Matteucci et a. (1981) J. Am. Chem. Soc. 103:3185). The resulting phosphite triester is finally oxidized to a phosphorotriester to complete the internucleotide bond (R. L. Letsinger et al. (1976) J. Am. Chem. Soc. 9:3655). Alternatively, the synthesis of phosphorothioate linkages can be carried out by sulfurization of the phosphite triester. Several chemicals can be used to perform this reaction, among them 3H-1,2-benzodithiole-3-one, 1,1-dioxide (R.P. Iyer, W. Egan, J.B. Regan, and S.L. Beaucage, J. Am. Chem. Soc., 1990, 112, 1253-1254). The steps of deprotection, coupling and oxidation are repeated until an oligonucleotide of the desired length and sequence is obtained. Other methods exist to generate oligonucleotides such as the H-phosphonate method (Hall et al, (1957) J. Chem. Soc., 3291-3296) or the phosphotriester method as described by Ikuta et al., Ann. Rev. Biochem. 53:323-356 (1984), (phosphotriester and phosphite-triester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994). Other forms of oligonucleotide synthesis are described in U.S. Patent No. 6,294,664 and U.S. Patent No. 6,291,669.

The nucleotide sequence of an oligonucleotide is generally determined by the sequential order in which subunits of subunit blocks are added to the oligonucleotide chain during synthesis. Each round of addition can involve a different, specific nucleotide precursor, or a mixture of one or more different nucleotide precursors. For the disclosed primers of specific sequence, specific nucleotide precursors would be added sequentially. In general, degenerate or random positions in an oligonucleotide can be produced by using a mixture of nucleotide precursors representing the range of nucleotides that can be present at that position. Thus, precursors for A and T can be included in the reaction for a particular position in an oligonucleotide if that position is to be degenerate for A and T. Precursors for all four nucleotides can be included for a fully degenerate or random position. Completely random oligonucleotides can be made by including all four nucleotide precursors in every round of synthesis. Degenerate oligonucleotides can also be made having different proportions of different nucleotides. Such oligonucleotides can be made, for example, by using different nucleotide precursors, in the desired proportions, in the reaction.

Many of the oligonucleotides described herein are designed to be complementary to certain portions of other oligonucleotides or nucleic acids such that stable hybrids can be formed between them. The stability of these hybrids can be calculated using known methods such as those described in Lesnick and Freier, Biochemistry 34:10807-10815 (1995), McGraw et al., Biotechniques 8:674-678 (1990), and Rychlik et al., Nucleic Acids Res. 18:6409-6412 (1990).

So long as their relevant function is maintained, primers, detection probes, address probes, and any other oligonucleotides can be made up of or include modified nucleotides (nucleotide analogs). Many modified nucleotides are known and can be used in oligonucleotides. A nucleotide analog is a nucleotide which contains some type of modification to either the base, sugar, or phosphate moieties. Modifications to the base moiety would include natural and synthetic modifications of A, C, G, and T/U as well as different purine or pyrimidine bases, such as uracil-5-yl, hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine-, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Additional base modifications can be found for example in U.S. Pat. No. 3,687,808, Englisch et al., Angewandte Chemie, International Edition, 1991, 30, 613, and Sanghvi, Y. S., Chapter 15, Antisense Research and Applications, pages 289-302, Crooke, S. T. and Lebleu, B. ed., CRC Press, 1993. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex formation. Other modified bases are those that function as universal bases. Universal bases include 3-nitropyrrole and 5-nitroindole. Universal bases substitute for the normal bases but have no bias in base pairing. That is, universal bases can base pair with any other base. Base modifications often can be combined with for example a sugar modification, such as 2'-O-methoxyethyl, to achieve unique properties such as increased duplex stability. There are numerous United States patents such as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, which detail and describe a range of base modifications.

Nucleotide analogs can also include modifications of the sugar moiety. Modifications to the sugar moiety would include natural modifications of the ribose and deoxyribose as well as synthetic modifications. Sugar modifications include but are not limited to the following modifications at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C1 to C10, alkyl or C2 to C10 alkenyl and alkynyl. 2' sugar modifications also include but are not limited to -O[(CH₂)n O]m CH₃, -O(CH₂)n OCH₃, -O(CH₂)n NH₂, - O(CH₂)n CH₃, -O(CH₂)n -ONH₂, and -O(CH₂)nON[(CH₂)n CH₃)]₂, where n and m are from 1 to about 10.

Other modifications at the 2' position include but are not limited to: C1 to C10 lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂ NO₂ N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs may also have sugar mimetics such as cyclobutyl moieties in place of the pentofuranosyl sugar. There are numerous United States patents that teach the preparation of such modified sugar structures such as 4,981,957; 5,118.800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkages between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included. Numerous United States patents teach how to make and use nucleotides containing modified phosphates and include but are not limited to, 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

It is understood that nucleotide analogs need only contain a single modification, but may also contain multiple modifications within one of the moieties or between different moieties.

Nucleotide substitutes are molecules having similar functional properties to nucleotides, but which do not contain a phosphate moiety, such as peptide nucleic acid (PNA). Nucleotide substitutes are molecules that will recognize and hybridize to (base pair to) complementary nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts. Numerous United States patents disclose how to make and use these types of phosphate replacements and include but are not limited to 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

It is also understood in a nucleotide substitute that both the sugar and the phosphate moieties of the nucleotide can be replaced, by for example an amide type linkage (aminoethylglycine) (PNA). United States patents 5,539,082; 5,714,331; and 5,719,262 teach how to make and use PNA molecules. (See also Nielsen et al., Science 254:1497-1500 (1991)).

Oligonucleotides can be comprised of nucleotides and can-be made up of different types of nucleotides or the same type of nucleotides. For example, one or more of the nucleotides in an oligonucleotide can be ribonucleotides, 2'-O-methyl ribonucleotides, or a mixture of ribonucleotides and 2'-O-methyl ribonucleotides; about 10% to about 50% of the nucleotides can be ribonucleotides, 2'-O-methyl ribonucleotides, or a mixture of ribonucleotides and 2'-O-methyl ribonucleotides; about 50% or more of the nucleotides can be ribonucleotides, 2'-O-methyl ribonucleotides, or a mixture of ribonucleotides and 2'-O-methyl ribonucleotides; or all of the nucleotides are ribonucleotides, 2'-O-methyl ribonucleotides, or a mixture of ribonucleotides and 2'-O-methyl ribonucleotides. Such oligonucleotides can be referred to as chimeric oligonucleotides.

### I. DNA polymerases

The DNA polymerase used in the disclosed methods is the Phi29 DNA polymerase. It is capable of displacing, either alone or in combination with a compatible strand displacement factor, a hybridized strand encountered during replication and is referred to herein as strand displacement DNA polymerases. A strand displacement DNA polymerase lacks a 5' to 3' exonuclease activity. Strand displacement is necessary to result in synthesis of multiple copies of a target sequence. A 5' to 3' exonuclease activity, if present, would result in the destruction of a synthesized strand. The DNA polymerases for use in the disclosed method is highly processive. The suitability of a DNA polymerase for use in the disclosed method can be readily determined by assessing its ability to carry out strand displacement replication. Particularly useful enzymes are able to displace nucleic acids in a length of at least 1000 bp. The polymerase can be heat labile or heat stabile. The polymerase can be a holoenzyme, a part of a holoenzyme, or a mutated or genetically modified form of polymerases from viruses, phages, prokaryotes, eukayrotes, or Archaebacteria. The polymerase can be isolated from the original organism or produced in a genetically modified organism.

The strand displacement DNA polymerase used is the bacteriophage φ29-type DNA polymerase (U.S. Patent Nos. 5,198,543 and 5,001,050 to Blanco et al.)*.*

As used herein, a thermolabile nucleic acid polymerase is a nucleic acid polymerase that is notably inactivated at the temperature at which an amplification reaction is carried out in the absence of an additive, dNTPs, and template nucleic acid. Thus, whether a nucleic acid polymerase is thermolabile depends on the temperature at which an amplification reaction is carried out. Note that as used herein, thermolability does not require denaturation or irreversible inactivation of a polymerase. All that is required is that the polymerase be notably incapable of performing template-dependent polymerization at the temperature at which an amplification reaction is carried out in the absence of an additive. Reversibly-inactivated polymerases can also be used.

As used herein, an elevated temperature is a temperature at or above which a given nucleic acid polymerase is notably inactivated in the absence of an additive, dNTPs, and template nucleic acid. Thus, what constitutes an elevated temperature depends on the particular nucleic acid polymerase. As used herein, notable inactivation refers to a reduction in activity of 40% or more. Substantial inactivation refers to a reduction in activity of 60% or more. Significant inactivation refers to a reduction in activity of 80% or more.

Strand displacement can be facilitated through the use of a strand displacement factor, such as helicase or single-stranded binding proteins. It is considered that any DNA polymerase that can perform strand displacement replication in the presence of a strand displacement factor is suitable for use in the disclosed method, even if the DNA polymerase does not perform strand displacement replication in the absence of such a factor. Strand displacement factors useful in strand displacement replication include BMRF1 polymerase accessory subunit (Tsurumi et al., J. Virology 67(12):7648-7653 (1993)), adenovirus DNA-binding protein (Zijderveld and van der Vliet, J. Virology 68(2):1158-1164 (1994)), herpes simplex viral protein ICP8 (Boehmer and Lehman, J. Virology 67(2):711-715 (1993); Skaliter and Lehman, Proc. Natl. Acad. Sci. USA 91(22):10665-10669 (1994)); single-stranded DNA binding proteins (SSB; Rigler and Romano, J. Biol. Chem. 270:8910-8919 (1995)); phage T4 gene 32 protein (Villemain and Giedroc, Biochemistry 35:14395-14404 (1996); and calf thymus helicase (Siegel et al., J. Biol. Chem. 267:13629-13635 (1992)).

The ability of a polymerase to carry out strand displacement replication can be determined by using the polymerase in a strand displacement replication assay such as described in the Example. The assay in the example can be modified as appropriate. Such assays should be performed at a temperature suitable for optimal activity for the enzyme being used, i.e. from 25-40°C for φ29 DNA polymerase.

### J. Kits

The materials described above can be packaged together in any suitable combination as a kit useful for performing the disclosed method. Kit components in a given kit can be designed and adapted for use together in the disclosed method. For example, disclosed are kits for amplifying circular genomes, the kit comprising at least a reaction buffer and a DNA polymerase. The components of such a kit are described elsewhere herein. In some forms of the disclosed kits, the kit can further comprise a set of primers. In some forms of the disclosed kits, the kit can further comprise a lysis solution. In some forms of the disclosed kits, the kit can further comprise a stabilization solution. In some forms of the disclosed kits, the kit can further comprise deoxynucleotide triphosphates. In some forms of the disclosed kits, the kit can further comprise one or more detection probes. Detection probes are described elsewhere herein. In some forms of the kit, the detection probes can each comprise a complementary portion, where the complementary portion is complementary to a nucleic acid sequence of interest. In some forms of the kit, the kit can further comprise denaturing solution. In some forms of the kit, the kit can further comprise reaction mix. The kits also can contain nucleotides, buffers, detection probes, fluorescent change probes, lysis solutions, stabilization solutions, denaturation solutions, or a combination.

Any of the components that can be present in a kit that can be used together can be combined in a single component of the kit. Thus, a reaction mix can include, for example, buffers, deoxynucleotide triphosphates and primers. Similarly, components and solutions can be divided into constituent parts or sub-solutions. The kits can be used for any purpose, generally for nucleic acid amplification. In some forms, the kit can be designed to detect nucleic acid sequences of interest in a genome or other nucleic acid sample. In some forms, the kit can be designed to assess a disease, condition or predisposition of an individual based on a nucleic acid sequences of interest.

### K. Mixtures

Disclosed are mixtures formed by performing, or formed during the course of performing, any form of the disclosed method. For example, disclosed are mixtures comprising, for example, a genomic nucleic acid sample, a set of primer primers, and DNA polymerase; a genomic nucleic acid sample, a set of primer primers, DNA polymerase, and tandem sequence DNA. Whenever the method involves mixing or bringing into contact, for example, compositions or components or reagents, performing the method creates a number of different mixtures. For example, if the method includes three mixing steps, after each one of these steps a unique mixture is formed if the steps are performed sequentially. In addition, a mixture is formed at the completion of all of the steps regardless of how the steps were performed. The present disclosure contemplates these mixtures, obtained by the performance of the disclosed method as well as mixtures containing any disclosed reagent, composition, or component, for example, disclosed herein.

### L. Systems

Disclosed are systems useful for performing, or aiding in the performance of, the disclosed method. Systems generally comprise combinations of articles of manufacture such as structures, machines, devices, and the like, and compositions, compounds, materials, and the like. Such combinations that are disclosed or that are apparent from the disclosure are contemplated. For example, disclosed and contemplated are systems comprising solid supports and primers, nucleic acid samples, detection probes, fluorescent change probes, or a combination.

### M. Data Structures and Computer Control

Disclosed are data structures used in, generated by, or generated from, the disclosed method. Data structures generally are any form of data, information, and/or objects collected, organized, stored, and/or embodied in a composition or medium. A nucleic acid library stored in electronic form, such as in RAM or on a storage disk, is a type of data structure.

The disclosed method, or any part thereof or preparation therefor, can be controlled, managed, or otherwise assisted by computer control. Such computer control can be accomplished by a computer controlled process or method, can use and/or generate data structures, and can use a computer program. Such computer control, computer controlled processes, data structures, and computer programs are contemplated and should be understood to be disclosed herein.

### Uses

The disclosed methods and compositions are applicable to numerous areas including, but not limited to, analysis of nucleic acids present in cells (for example, analysis of genomic DNA in cells), disease detection, mutation detection, gene discovery, gene mapping (molecular haplotyping), and agricultural research.

### Method

Disclosed is a method for amplification of circular genomes as defined in claim 1. The method is based on rolling circle amplification of the circular genomes which involves strand displacement replication by primers. The disclosed method allows differential amplification of circular genomes of interest. In genomic nucleic acid samples containing both a circular genome of interest and non-target nucleic acids, such as non-target genomes, the disclosed methods and compositions can result in many-fold differential amplification of the circular genome of interest over non-target nucleic acids. It has been discovered that selection of a set of primers complementary to a circular genome of interest can result in much greater amplification of the circular genome of interest relative to non-target nucleic acids present. Such differential amplification of circular genomes is very useful for obtaining useful amounts of genomes of interest from a mixed nucleic acid sample. For example, mitochondrial genomes, which, absent complicated and time consuming purification, are in the presence of non-target nucleic acids (such as the host cell genome), can be differentially amplified relative to the host cell genome and other non-target nucleic acids using the disclosed methods and composition.

The disclosed methods involves bringing into contact a set of primers, DNA polymerase, and a genomic nucleic acid sample, where the genomic nucleic acid sample comprises a circular genome, and incubating the genomic nucleic acid sample under conditions that promote replication of the circular genome in the genomic nucleic acid sample. Replication of the circular genome can proceed by rolling circle replication. The conditions that promote replication of the circular genome does not involve thermal cycling and can be substantially isothermic. In the disclosed methods, the circular genome is differentially replicated compared to the non-target nucleic acids present in the genomic nucleic acid sample. Thus, for example, non-target nucleic acids present in the genomic nucleic acid sample generally would not be substantially, significantly, or notably replicated. For example, the primers in the set of primers and the reaction conditions generally can be selected such that non-target nucleic acids in the genomic nucleic acid sample are not substantially, significantly, or notably replicated.

Replication of the circular genomes results in replicated strands. Such replication proceeds by rolling circle replication to produce tandem sequence DNA. The replicated strands are displaced from the nucleic acid molecules by strand displacement replication of another replicated strand. Such amplification can proceed by replication with a highly processive polymerase initiating at each primer and continuing until spontaneous termination. A useful feature of the method is that as a DNA polymerase extends a primer, the polymerase displaces the replication products (that is, DNA strands) that resulted from extension of other primers. The polymerase is continuously extending new primers and displacing the replication products of previous priming events. In this way, multiple overlapping copies of all of the nucleic acid molecules and sequences in the circular genome can be synthesized in a short time. The disclosed method has advantages over the polymerase chain reaction since it can be carried out under isothermal conditions. In the disclosed method amplification takes place not in cycles, but in a continuous, isothermal replication. This makes amplification less complicated and much more consistent in output. Strand displacement allows rapid generation of multiple copies of a nucleic acid sequence or sample in a single, continuous, isothermal reaction.

When the genomic nucleic acid sample comprises one or more non-target genomes (including non-target genomes that may be circular), the circular genome of interest can be differentially amplified relative to the non-target genomes. The genomic nucleic acid sample can comprises a plurality of genomes including the circular genome of interest and one or more non-target genomes. Thus, for example, non-target nucleic acids present in the genomic nucleic acid sample generally would not be substantially, significantly, or notably replicated and/or amplified. For example, the primers in the set of primers and the reaction conditions generally can be selected such that non-target nucleic acids in the genomic nucleic acid sample are not substantially, significantly, or notably replicated and/or amplified.

The differential amplification of the circular genome can be described in quantitative terms. For example, the circular genome can be amplified, for example, at least 10 fold, 20 fold, 30 fold, 40 fold, 50 fold, 60 fold, 75 fold, 100 fold, 150 fold, 200 fold, 500 fold, 1000 fold, 2000 fold, 5000 fold, 10000 fold, 20000 fold, 50000 fold, 100000 fold, 200000 fold, 500000 fold, 1000000 fold, or 2000000 fold or more compared to the non-target nucleic acids and/or non-target genomes. Such differential amplification generally can be assessed by measuring the relative amplification of selected sequences within the circular genome and within one or more non-target nucleic acids and/or non -target genomes. Such assessments are described elsewhere herein.

The non-target genomes can be any genome that may be in a genomic nucleic acid sample. For example, non-target genomes can be, for example, bacterial genomes, viral genomes, microbial genomes, pathogen genomes, eukaryotic genomes, plant genomes, animal genomes, vertebrate genomes, fish genomes, avian genomes, mammalian genomes, rodent genomes, murine genomes, human genomes, host genomes, and/or non-target circular genomes. Circular genomes can be in the presence of non-target genomes. For example, organelle genomes are commonly in the presence of the cell genome of the cell in which the organelle resides. Pathogen genomes are commonly in the presence of host cell genomes.

Any material or sample containing or suspected of containing a circular genome of interest can be used as the genomic nucleic acid sample. For example, the genomic nucleic acid sample can be a blood sample, a urine sample, a semen sample, a lymphatic fluid sample, a cerebrospinal fluid sample, amniotic fluid sample, a biopsy sample, a needle aspiration biopsy sample, a cancer sample, a tumor sample, a tissue sample, a cell sample, a cell lysate sample, a crude cell lysate sample, a forensic sample, an archeological sample, an infection sample, a nosocomial infection sample, an environmental sample, or a combination thereof

Also disclosed is a method of identifying a set of primers for differential amplification of a circular genome. Such a method can generally involve selecting test primers for a test set of primers, bringing into contact the test set of primers, DNA polymerase, and a genomic nucleic acid sample, incubating the genomic nucleic acid sample under conditions that promote replication of the circular genome in the genomic nucleic acid sample, and determining the relative amplification of the circular genome and non-target nucleic acids. The test set of primers are identified if the circular genome is amplified at least 10 fold compared to the non-target nucleic acids. Generally, each primer can specifically hybridize to a nucleotide sequence in a circular genome of interest such that the distance between consecutive primers hybridized to the same strand of the circular genome averages from, for example, about 200 to about 20000 nucleotides, or about 200 to about 6000 nucleotides. The test genomic nucleic acid sample comprises the circular genome and non-target nucleic acids. Replication of the circular genome proceeds by rolling circle replication. Generally, the conditions that promote replication of the circular genome do not involve thermal cycling and/or can be substantially isothermic.

Following amplification, the amplified sequences can be used for any purpose, such as uses known and established for amplified sequences. For example, amplified sequences can be detected using any of conventional detection systems for nucleic acids such as detection of fluorescent labels, enzyme-linked detection systems, antibody-mediated label detection, and detection of radioactive labels. A preferred form of labeling involves labeling of the replicated strands (that is, the strands produced in multiple displacement amplification) using terminal deoxynucleotidyl transferase. The replicated strands can be labeled by, for example, the addition of modified nucleotides, such as biotinylated nucleotides, fluorescent nucleotides, 5 methyl dCTP, BrdUTP, or 5-(3-aminoallyl)-2'-deoxyuridine 5'-triphosphates, to the 3' ends of the replicated strands. Amplification of forensic material for RFLP-based testing is one useful application for the disclosed method.

Non-target nucleic acids present in the genomic nucleic acid sample generally would not be substantially, significantly, and/or notably replicated and/or amplified in the disclosed methods. As used herein, a substantial replication or amplification of a nucleic acid refers to an increase in the amount of the nucleic acid of 50 fold or more. As used herein, a notable replication or amplification of a nucleic acid refers to an increase in the amount of the nucleic acid of 10 fold or more. As used herein, a significant replication or amplification of a nucleic acid refers to an increase in the amount of the nucleic acid of 5 fold or more.

Nucleotides incorporated during Rolling Circle Amplification may be dNTPs, NTPs, modified by tags having a molecular weight larger than a methyl group such as fluorophores, haptens etc. Many such modified nucleotides for direct incorporation into replicated nucleic acids are known and can be use in the disclosed method.

The disclosed amplification generally is carried out in the presence of a buffer. The buffer can be a solution that contains components needed to provide solution conditions that promote replication of the circular genome. The pH of the buffer is 8.5 and 9. Ions dissociated in the buffer may help to increase the reaction product. The buffer may contain Mg²⁺ ions in a concentration between 0.1 mM and 30 mM, preferably between 0.1 mM and 25 mM, more preferably between 1 mM and 20 mM, most preferably between 2 mM and 15 mM. The buffer may contain monovalent salts such as NaCl or KCl in a concentration between 0.1 mM and 200 mM, preferably between 1 mM and 150 mM, more preferably between 5 mM and 125 mM, most preferably between 10 mM and 100 mM. The buffer may contain ammonia salts such as (NH₄)₂SO₄, or NH₄Cl, or salts of polyamines such as spermine, spermidine, amino acids. For example, the buffer can comprise Tris, pH 8.5; 10 mM MgCl₂, 50 mM KCI, 20 mM (NH₄)₂SO₄.

The disclosed amplification can be carried out at any suitable temperature that allows replication of the circular genome. For example, the reaction temperature can be, for example, between 20 and 80°C, between 22°C and 70°C, between 25°C and 47°C, or between 25°C and 42°C.

The conditions that promote replication of the circular genome do not involve thermal cycling and can be substantially isothermic. The temperature during amplification reaction can be substantially isothermal. As used herein, substantially isothermal means that the reaction temperature does not change during amplification of nucleic acids by more than 10%, excluding initial and/or terminal heating and cooling (such heating and cooling could be used for denaturation, hybridization and/or enzyme inactivation or activation). The amplification can be carried out at a reaction temperature that does not change by more than 25%, 15%, 10%, 9%, 8%, 7%, 6%, 5%, 4%, 3%, 2%, or 1% during the amplification. The amplification can be carried out in the absence of thermal cycling, excluding initial and/or terminal heating and cooling. Thus, for example, the amplification can be carried out with no more than 4, no more than 3, or no more than 2 significant temperature changes. A significant temperature change is a temperature change of greater than 10%. The amplification can be carried out with no more than 4, no more than 3, or no more than 2 changes in reaction temperature of more than 25%, 15%, 10%, 9%, 8%, 7%, 6%, 5%, 4%, 3%, 2%, or 1% during the amplification.

The concentration of nucleic acids in the amplification mixture can be, for example, 300 ng/µl or less, 200 ng/µl or less, 150 ng/µl or less, 100 ng/µl or less, 95 ng/µl or less, 90 ng/µl or less, 85 ng/µl or less, 80 ng/µl or less, 75 ng/µl or less, 70 ng/µl or less, 65 ng/µl or less, 60 ng/µl or less, 55 ng/µl or less, 50 ng/µl or less, 45 ng/µl or less, 40 ng/µl or less, 35 ng/µl or less, 30 ng/µl or less, 25 ng/µl or less, 20 ng/µl or less, 15 ng/µl or less, 10 ng/µl or less, 9 ng/µl or less, 8 ng/µl or less, 7 ng/µl or less, 6 ng/µl or less, 5 ng/µl or less, 4 ng/µl or less, 3 ng/µl or less, 2 ng/µl or less, 1 ng/µl or less, 0.8 ng/µl or less, 0.6 ng/µl or less, 0.5 ng/µl or less, 0.4 ng/µl or less, 0.3 ng/µl or less, 0.2 ng/µl or less, or 0.1 ng/µl or less.

The disclosed method involves rolling circle amplification. Rolling circle amplification refers to nucleic acid amplification reactions where a circular nucleic acid template is replicated in a single long strand with tandem repeats of the sequence of the circular template. This first, directly produced tandem repeat strand is referred to as tandem sequence DNA (TS-DNA) and its production is referred to as rolling circle replication. Rolling circle amplification refers both to rolling circle replication and to processes involving both rolling circle replication and additional forms of amplification. For example, tandem sequence DNA can be replicated to form complementary strands referred to a secondary tandem sequence DNA. Secondary tandem sequence DNA can, in turn, be replicated, and so on. Tandem sequence DNA can also be transcribed. Rolling circle amplification involving production of only the first tandem sequence DNA (that is, the replicated strand produced by rolling circle replication) can be referred to as of linear rolling circle amplification (where "linear" refers to the general amplification kinetics of the amplification).

### A. Amplification Level

The disclosed method can produce a high level of amplification. For example, the disclosed method can produce a 10,000-fold amplification or more. Fold amplification refers to the number of copies generated of the template being amplified. For example, if I µg of DNA is generated from 1 ng of template, the level of amplification is 1,000-fold. The disclosed method can produce, for example, amplification of about 1-fold, about 2-fold, about 3-fold, about 4-fold, about 5-fold, about 6-fold, about 7-fold, about 8-fold, about 9-fold, about 10-fold, about 11-fold, about 12-fold, about 14-fold, about 16-fold, about 20-fold, about 24-fold, about 30-fold, about 35-fold, about 40-fold, about 50-fold, about 60-fold, about 70-fold, about 80-fold, about 90-fold, about 100-fold, about 150-fold, about 200-fold, about 250-fold, about 300-fold, about 400-fold, about 500-fold, about 600-fold, about 700-fold, about 800-fold, about 900-fold, about 1,000-fold, about 10,000-fold, about 100,000-fold, about 1,000,000-fold, about 10,000,000-fold, or about 100,000,000-fold.

The disclosed method can produce, for example, amplification of at least 2-fold, at least 3-fold, at least 4-fold, at least 5-fold, at least 6-fold, at least 7-fold, at least 8-fold, at least 9-fold, at least 10-fold, at least 11-fold, at least 12-fold, at least 14-fold, at least 16-fold, at least 20-fold, at least 24-fold, at least 30-fold, at least 35-fold, at least 40-fold, at least 50-fold, at least 60-fold, at least 70-fold, at least 80-fold, at least 90-fold, at least 100-fold, at least 150-fold, at least 200-fold, at least 250-fold, at least 300-fold, at least 400-fold, at least 500-fold, at least 600-fold, at least 700-fold, at least 800-fold, at least 900-fold, at least 1,000-fold, at least 10,000-fold, at least 100,000-fold, at least 1,000,000-fold, at least 10,000,000-fold, or at least 100,000,000-fold.

### B. Primer Selection

Primers and sets of primers for use in the disclosed method can be selected for their ability to differentially amplify a circular genome of interest to particular desired extents. Such primers and sets of primers are particularly useful in the disclosed method. All of the primers can be selected primers or some of the primers can be selected primers. Any useful criteria can be used for primer selection. Useful criteria include the relative amplification of the circular genome compared to non-target nucleic acids and the level of amplification of the circular genome. Primers and sets of primers that meet given selection criteria (or a selection criterion) are referred to herein as selected primers and selected primer sets (for those selection criteria). Primers and sets of primers that do not meet the given selection criteria (or selection criterion) are referred to herein as non-selected primers and non-selected primer sets (for those selection criteria). Both selected and non-selected primers can be used together in the disclosed method, although use of selected primers is preferred.

Selected primers and sets of primers meeting different selection criteria can be used together in the disclosed method. That is, the primers and sets of primers used in a given amplification reaction need not all have the same capabilities or meet the same criteria. Similarly, non-selected primers and non-selected primer sets failing to meet different selection criteria can be included or excluded from use in the disclosed method. That is, primers and sets of primers not used (or used) need not lack the same capabilities or fail to meet the same criteria. Selected primers and sets of primers meeting a selection criterion, selection criteria, or a combination of different selection criteria, can be used with non-selected primers and non-selected primer sets failing to meet the same or a different selection criterion, selection criteria, or a combination of the same or different selection criteria.

The disclosed method thus can be performed with one or more selected primers or sets of primers. The disclosed method can also be performed with one or more selected primers or sets of primers and one or more non-selected primers or non-selected primer sets. Whether a primer or primer set is a selected primer or primer set or a non-selected primer or primer set can be determined by testing the primer or primer set for the selection criterion or criteria. Thus, for example, the primer or primer set can be tested in a form of the disclosed method. Such a method could use a nucleic acid sample of interest, such as a nucleic acid sample having relevant characteristics. A nucleic acid sample used for this purpose is referred to herein as a selection nucleic acid sample. Particularly useful selection nucleic acid samples are nucleic acid samples of the same type that the selected primers will be used to amplify. Thus, a human nucleic acid sample can be used as the selection sample for selecting primers to be used to amplify human mitochondrial DNA.

A primer or primer set can be selected based on producing a certain level or range of differential amplification of circular genome relative to non-target nucleic acids in a selection nucleic acid sample. Any amplification level can be used. For example, any of the amplification levels described elsewhere herein can be used as the selection criterion. A selected primer or primer set can produce, for example, differential amplification of about 1-fold, about 2-fold, about 3-fold, about 4-fold, about 5-fold, about 6-fold, about 7-fold, about 8-fold, about 9-fold, about 10-fold, about 11-fold, about 12-fold, about 14-fold, about 16-fold, about 20-fold, about 24-fold, about 30-fold, about 35-fold, about 40-fold, about 50-fold, about 60-fold, about 70-fold, about 80-fold, about 90-fold, about 100-fold, about 150-fold, about 200-fold, about 250-fold, about 300-fold, about 400-fold, about 500-fold, about 600-fold, about 700-fold, about 800-fold, about 900-fold, about 1,000-fold, about 10,000-fold, about 100,000-fold, about 1,000,000-fold, about 10,000,000-fold, or about 100,000,000-fold. Fold amplification refers to the number of copies generated of the template being amplified. For example, if 1 µg of DNA is generated from 1 ng of template, the level of amplification is 1,000-fold.

A selected primer or primer set can produce, for example, differential amplification of at least 2-fold, at least 3-fold, at least 4-fold, at least 5-fold, at least 6-fold, at least 7-fold, at least 8-fold, at least 9-fold, at least 10-fold, at least 11-fold, at least 12-fold, at least 14-fold, at least 16-fold, at least 20-fold, at least 24-fold, at least 30-fold, at least 35-fold, at least 40-fold, at least 50-fold, at least 60-fold, at least 70-fold, at least 80-fold, at least 90-fold, at least 100-fold, at least 150-fold, at least 200-fold, at least 250-fold, at least 300-fold, at least 400-fold, at least 500-fold, at least 600-fold, at least 700-fold, at least 800-fold, at least 900-fold, at least 1,000-fold, at least 10,000-fold, at least 100,000-fold, at least 1,000,000-fold, at least 10,000,000-fold, or at least 100,000,000-fold.

### C. Nucleic Acid Sample Preparation and Treatment

Nucleic acids for amplification are often obtained from cellular samples. This generally requires disruption of the cell (to make the nucleic acid accessible) and can include purification of the nucleic acids prior to amplification. It also can include the separation of nucleic acids and other biomolecules. It also can include the inactivation of protein factors such as nucleases that could degrade the DNA, or of factors such as histones that could bind to DNA strands and impede their use as a template for DNA synthesis by a polymerase. It also can include the use of enzymes to degrade biomolecules that contaminate the nucleic acid sample. These enzymes can comprise Proteases (e.g. Proetase K, Trypsin, Chymotrypsin, Collagenase, or other enzymes deemed to be suitable by the artisan). These enzymes can comprise Nucleases (.e.g. RNases, Exonucleases). These enzymes may comprise single-stranded specific nucleases if dsDNA is the circular genome of interest. These enzymes can comprise Polysaccharide degrading enzymes (e.g. Hyalurondiase, amylase, pectinase, cellulose) These enzymes may comprise lipases (e.g. pancreatic lipase) There are a variety of techniques used to break open cells, such as sonication, freeze-thaw cycles, enzymatic digestion of cell walls, heating, and exposure to lytic conditions (e.g. organis solvents, chaotropic salts, hypothonic conditions). Some lytic conditions involve use of non-physiological pH and/or solvents. Many lytic techniques can result in damage to nucleic acids in cells, including, for example, breakage of genomic DNA. In particular, use of heating to lyse cells can damage genomic DNA and reduce the amount and quality of amplification products of genomic DNA. It has been discovered that alkaline lysis can cause less damage to genomic DNA and can thus result in higher quality amplification results. Alkaline lysis also inactivates protein factors such as nucleases, histones, or other factors that could impede the amplification of DNA within the sample. In addition, it is a useful property of alkaline lysis that reducing the pH does not reactivate the protein factors, but that such protein factors remain inactivated when the pH of the solution is brought back within a neutral range.

In some forms of the disclosed method, a genomic sample is prepared by exposing cells to alkaline conditions, thereby lysing the cells and resulting in a cell lysate; reducing the pH of the cell lysate to make the pH of the cell lysate compatible with DNA replication; and incubating the cell lysate under conditions that promote replication of the genome of the cells by multiple displacement amplification. Alkaline conditions are conditions where the pH is greater than 9.0. Particularly useful alkaline conditions for the disclosed method are conditions where the pH is greater than 10.0. The alkaline conditions can be, for example, those that cause a substantial number of cells to lyse, those that cause a significant number of cells to lyse, or those that cause a sufficient number of cells to lyse. The number of lysed cells can be considered sufficient if the genome can be sufficiently amplified in the disclosed method. The amplification is sufficient if enough amplification product is produced to permit some use of the amplification product, such as detection of sequences or other analysis. The reduction in pH is generally into the neutral range of pH 9.0 to pH 8.5.

The cells can be exposed to alkaline conditions by mixing the cells with a lysis solution. The amount of lysis solution mixed with the cells can be that amount that causes a substantial number of cells to lyse or those that cause a sufficient number of cells to lyse. Generally, this volume will be a function of the pH of the cell/lysis solution mixture. Thus, the amount of lysis solution to mix with cells can be determined generally from the volume of the cells and the alkaline concentration of the lysis buffer. For example, a smaller volume of a lysis solution with a stronger base and/or higher concentration of base would be needed to create sufficient alkaline conditions than the volume needed of a lysis solution with a weaker base and/or lower concentration of base. The lysis solution can be formulated such that the cells are mixed with an equal volume of the lysis solution (to produce the desired alkaline conditions).

In some embodiments, the lysis solution can comprise a base, such as an aqueous base. Useful bases include potassium hydroxide, sodium hydroxide, potassium acetate, sodium acetate, ammonium hydroxide, lithium hydroxide, calcium hydroxide, magnesium hydroxide, sodium carbonate, sodium bicarbonate, calcium carbonate, ammonia, aniline, benzylamine, n-butylamine, diethylamine, dimethylamine, diphenylamine, ethylamine, ethylenediamine, methylamine, N-methylaniline, morpholine, pyridine, triethylamine, trimethylamine, aluminum hydroxide, rubidium hydroxide, cesium hydroxide, strontium hydroxide, barium hydroxide, and DBU (1,8-diazobicyclo[5,4,0]undec-7-ene). Useful formulations of lysis solution include lysis solution comprising 400 mM KOH, lysis solution comprising 400 mM KOH, 100 mM dithiothreitol, and 10 mM EDTA, and lysis solution consisting of 400 mM KOH, 100 mM dithiothreitol, and 10 mM EDTA.

In some embodiments, the lysis solution can comprise a plurality of basic agents. As used herein, a basic agent is a compound, composition or solution that results in alkaline conditions. In some embodiments, the lysis solution can comprise a buffer. Useful buffers include phosphate buffers, "Good" buffers (such as BES, BICINE, CAPS, EPPS, HEPES, MES, MOPS, PIPES, TAPS, TES, and TRICINE), sodium cacodylate, sodium citrate, triethylammonium acetate, triethylammonium bicarbonate, Tris, Bis-tris, and Bis-tris propane. The lysis solution can comprise a plurality of buffering agents. As used herein, a buffering agent is a compound, composition or solution that acts as a buffer. An alkaline buffering agent is a buffering agent that results in alkaline conditions. In some embodiments, the lysis solution can comprise a combination of one or more bases, basic agents, buffers and buffering agents.

The pH of the cell lysate can be reduced to form a stabilized cell lysate. A stabilized cell lysate is a cell lysate the pH of which is in the neutral range (from about pH 6.0 to about pH 9.0). Useful stabilized cell lysates have a pH that allows replication of nucleic acids in the cell lysate. For example, the pH of the stabilized cell lysate is usefully at a pH at which the DNA polymerase can function. The pH of the cell lysate can be reduced by mixing the cell lysate with a stabilization solution. The stabilization solution comprises a solution that can reduce the pH of a cell lysate exposed to alkaline conditions as described elsewhere herein.

The amount of stabilization solution mixed with the cell lysate can be that amount that causes a reduction in pH to the neutral range (or other desired pH value). Generally, this volume will be a function of the pH of the cell lysate/stabilization solution mixture. Thus, the amount of stabilization solution to mix with the cell lysate can be determined generally from the volume of the cell lysate, its pH and buffering capacity, and the acidic concentration of the stabilization buffer. For example, a smaller volume of a stabilization solution with a stronger acid and/or higher concentration of acid would be needed to reduce the pH sufficiently than the volume needed of a stabilization solution with a weaker acid and/or lower concentration of acid. The stabilization solution can be formulated such that the cell lysate is mixed with an equal volume of the stabilization solution (to produce the desired pH).

In some embodiments, the stabilization solution can comprise an acid. Useful acids include hydrochloric acid, sulfuric acid, phosphoric acid, acetic acid, acetylsalicylic acid, ascorbic acid, carbonic acid, citric acid, formic acid, nitric acid, perchloric acid, HF, HBr, HI, H₂S, HCN, HSCN, HClO, monochloroacetic acid, dichloroacetic acid, trichloroacetic acid, and any carboxylic acid (ethanoic, propanoic, butanoic, etc., including both linear or branched chain carboxylic acids). In some embodiments, the stabilization solution can comprise a buffer. Useful buffers include Tris-HCl, HEPES, "Good" buffers (such as BES, BICINE, CAPS, EPPS, HEPES, MES, MOPS, PIPES, TAPS, TES, and TRICINE), sodium cacodylate, sodium citrate, triethylammonium acetate, triethylammonium bicarbonate, Tris, Bis-tris, and Bis-tris propane. Useful formulations of stabilization solutions include stabilization solution comprising 800 mM Tris-HCl; stabilization solution comprising 800 mM Tris-HCl at pH 4.1; and stabilization solution consisting of 800 mM Tris-HCl, pH 4.1.

In some embodiments, the stabilization solution can comprise a plurality of acidic agents. As used herein, an acidic agent is a compound, composition or solution that forms an acid in solution. In some embodiments, the stabilization solution can comprise a plurality of buffering agents. An acidic buffering agent is a buffering agent that forms an acid in solution. In some embodiments, the stabilization solution can comprise a combination of one or more acids, acidic agents, buffers and buffering agents.

In some embodiments, the cells are not lysed by heat. Those of skill in the art will understand that different cells under different conditions will be lysed at different temperatures and so can determine temperatures and times at which the cells will not be lysed by heat. In general, the cells are not subjected to heating above a temperature and for a time that would cause substantial cell lysis in the absence of the alkaline conditions used. As used herein, substantial cell lysis refers to lysis of 90% or greater of the cells exposed to the alkaline conditions. Significant cell lysis refers to lysis of 50% or more of the cells exposed to the alkaline conditions. Sufficient cell lysis refers to lysis of enough of the cells exposed to the alkaline conditions to allow synthesis of a detectable amount of amplification products by multiple strand displacement amplification. In general, the alkaline conditions used in the disclosed method need only cause sufficient cell lysis. It should be understood that alkaline conditions that could cause significant or substantial cell lysis need not result in significant or substantial cell lysis when the method is performed.

In some embodiments, the cells are not subjected to heating substantially or significantly above the temperature at which the cells grow. As used herein, the temperature at which the cells grow refers to the standard temperature, or highest of different standard temperatures, at which cells of the type involved are cultured. In the case of animal cells, the temperature at which the cells grow refers to the body temperature of the animal. In other embodiments, the cells are not subjected to heating substantially or significantly above the temperature of the amplification reaction (where the genome is replicated).

In some embodiments, the cell lysate is not subjected to purification prior to the amplification reaction. In the context of the disclosed method, purification generally refers to the separation of nucleic acids from other material in the cell lysate. It has been discovered that multiple displacement amplification can be performed on unpurified and partially purified samples. It is commonly thought that amplification reactions cannot be efficiently performed using unpurified nucleic acid. In particular, PCR is very sensitive to contaminants.

Forms of purification include centrifugation, extraction, chromatography, precipitation, filtration, and dialysis. Partially purified cell lysate includes cell lysates subjected to centrifugation, extraction, chromatography, precipitation, filtration, and dialysis. Partially purified cell lysate generally does not include cell lysates subjected to nucleic acid precipitation or dialysis. As used herein, separation of nucleic acid from other material refers to physical separation such that the nucleic acid to be amplified is in a different container or container from the material. Purification does not require separation of all nucleic acid from all other materials. Rather, what is required is separation of some nucleic acid from some other material. As used herein in the context of nucleic acids to be amplified, purification refers to separation of nucleic acid from other material. In the context of cell lysates, purification refers to separation of nucleic acid from other material in the cell lysate. As used herein, partial purification refers to separation of nucleic acid from some, but not all, of other material with which the nucleic acid is mixed. In the context of cell lysates, partial purification refers to separation of nucleic acid from some, but not all, of the other material in the cell lysate.

Unless the context clearly indicates otherwise, reference herein to a lack of purification, lack of one or more types of purification or separation operations or techniques, or exclusion of purification or one or more types of purification or separation operations or techniques does not encompass the exposure of cells to alkaline conditions (or the results thereof) the reduction of pH of a cell lysate (or the results thereof). That is, to the extent that the alkaline conditions and pH reduction of the disclosed method produce an effect that could be considered "purification" or "separation," such effects are excluded from the definition of purification and separation when those terms are used in the context of processing and manipulation of cell lysates and stabilized cell lysates (unless the context clearly indicates otherwise).

As used herein, substantial purification refers to separation of nucleic acid from at least a substantial portion of other material with which the nucleic acid is mixed. In the context of cell lysates, substantial purification refers to separation of nucleic acid from at least a substantial portion of the other material in the cell lysate. A substantial portion refers to 90% of the other material involved. Specific levels of purification can be referred to as a percent purification (such as 95% purification and 70% purification). A percent purification refers to purification that results in separation from nucleic acid of at least the designated percent of other material with which the nucleic acid is mixed.

Denaturation of nucleic acid molecules to be amplified is common in amplification techniques. This is especially true when amplifying genomic DNA. In particular, PCR uses multiple denaturation cycles. Denaturation is generally used to make nucleic acid strands accessible to primers. Nucleic acid molecules, genomic DNA, for example, need not be denatured for efficient multiple displacement amplification. Elimination of a denaturation step and denaturation conditions has additional advantages such as reducing sequence bias in the amplified products (that is, reducing the amplification bias). In preferred forms of the disclosed method, the nucleic acid sample or template nucleic acid is not subjected to denaturating conditions and/or no denaturation step is used.

In some forms of the disclosed method, the nucleic acid sample or template nucleic acid is not subjected to heat denaturating conditions and/or no heat denaturation step is used. In some forms of the disclosed method, the nucleic acid sample or template nucleic acid is not subjected to alkaline denaturating conditions and/or no alkaline denaturation step is used. It should be understood that while sample preparation (for example, cell lysis and processing of cell extracts) may involve conditions that might be considered denaturing (for example, treatment with alkali), the denaturation conditions or step eliminated in some forms of the disclosed method refers to denaturation steps or conditions intended and used to make nucleic acid strands accessible to primers. Such denaturation is commonly a heat denaturation, but can also be other forms of denaturation such as chemical denaturation. It should be understood that in the disclosed method where the nucleic acid sample or template nucleic acid is not subjected to denaturing conditions, the template strands are accessible to the primers (since amplification occurs). However, the template stands are not made accessible via general denaturation of the sample or template nucleic acids.

Alternatively, the nucleic acid sample or template nucleic acid can be subjected to denaturating conditions and/or a denaturation step can be used. In some forms of the disclosed method, the nucleic acid sample or template nucleic acid can be subjected to heat denaturating conditions and/or a heat denaturation step can be used. In some forms of the disclosed method, the nucleic acid sample or template nucleic acid can be subjected to alkaline denaturating conditions and/or an alkaline denaturation step can be used.

### D. Detection of Amplification Products

Products of amplification can be detected using any nucleic acid detection technique. For real-time detection, the amplification products and the progress of amplification are detected during amplification. Real-time detection is usefully accomplished using one or more or one or a combination of fluorescent change probes and fluorescent change primers. Other detection techniques can be used, either alone or in combination with real-timer detection and/or detection involving fluorescent change probes and primers. Many techniques are known for detecting nucleic acids. The nucleotide sequence of the amplified sequences also can be determined using any suitable technique.

### E. Modifications And Additional Operations

### 1. Detection of Amplification Products

Amplification products can be detected directly by, for example, primary labeling or secondary labeling, as described below.

### i. Primary Labeling

Primary labeling consists of incorporating labeled moieties, such as fluorescent nucleotides, biotinylated nucleotides, digoxygenin-containing nucleotides, or bromodeoxyuridine, during strand displacement replication. For example, one may incorporate cyanine dye deoxyuridine analogs (Yu et al., Nucleic Acids Res., 22:3226-3232 (1994)) at a frequency of 4 analogs for every 100 nucleotides. A preferred method for detecting nucleic acid amplified *in situ* is to label the DNA during amplification with BrdUrd, followed by binding of the incorporated BrdU with a biotinylated anti-BrdU antibody (Zymed Labs, San Francisco, CA), followed by binding of the biotin moieties with Streptavidin-Peroxidase (Life Sciences, Inc.), and finally development of fluorescence with Fluorescein-tyramide (DuPont de Nemours & Co., Medical Products Dept.). Other methods for detecting nucleic acid amplified *in situ* include labeling the DNA during amplification with 5-methylcytosine, followed by binding of the incorporated 5-methylcytosine with an antibody (Sano et al., Biochim. Biophys. Acta 951:157-165 (1988)), or labeling the DNA during amplification with aminoallyl-deoxyuridine, followed by binding of the incorporated aminoallyl-deoxyuridine with an Oregon Green® dye (Molecular Probes, Eugene, OR) (Henegariu et al., Nature Biotechnology 18:345-348 (2000)).

Another method of labeling amplified nucleic acids is to incorporate 5-(3-aminoallyl)-dUTP (AAdUTP) in the nucleic acid during amplification followed by chemical labeling at the incorporated nucleotides. Incorporated 5-(3-aminoallyl)-deoxyuridine (AAdU) can be coupled to labels that have reactive groups that are capable of reacting with amine groups. AAdUTP can be prepared according to Langer et al. (1981). Proc. Natl. Acad. Sci. USA. 78: 6633-37. Other modified nucleotides can be used in analogous ways. That is, other modified nucleotides with minimal modification can be incorporated during replication and labeled after incorporation.

Examples of labels suitable for addition to AAdUTP are radioactive isotopes, fluorescent molecules, phosphorescent molecules, enzymes, antibodies, and ligands. Examples of suitable fluorescent labels include fluorescein isothiocyanate (FITC), 5,6-carboxymethyl fluorescein, Texas red, nitrobenz-2-oxa-1,3-diazol-4-yl (NBD), coumarin, dansyl chloride, rhodamine, amino-methyl coumarin (AMCA), Eosin, Erythrosin, BODIPY^{®}, Cascade Blue^{®}, Oregon Green^{®}; pyrene, lissamine, xanthenes, acridines, oxazines, phycoerythrin, macrocyclic chelates of lanthanide ions such as quantum dye^{™}, fluorescent energy transfer dyes, such as thiazole orange-ethidium heterodimer, and the cyanine dyes Cy3, Cy3.5, Cy5, Cy5.5 and Cy7. Examples of other specific fluorescent labels include 3-Hydroxypyrene 5,8,10-Tri Sulfonic acid, 5-Hydroxy Tryptamine (5-HT), Acid Fuchsin, Alizarin Complexon, Alizarin Red, Allophycocyanin, Aminocoumarin, Anthroyl Stearate, Astrazon Brilliant Red 4G, Astrazon Orange R, Astrazon Red 6B, Astrazon Yellow 7 GLL, Atabrine, Auramine, Aurophosphine, Aurophosphine G, BAO 9 (Bisaminophenyloxadiazole), BCECF, Berberine Sulphate, Bisbenzamide, Blancophor FFG Solution, Blancophor SV, Bodipy F1, Brilliant Sulphoflavin FF, Calcien Blue, Calcium Green, Calcofluor RW Solution, Calcofluor White, Calcophor White ABT Solution, Calcophor White Standard Solution, Carbostyryl, Cascade Yellow, Catecholamine, Chinacrine, Coriphosphine O, Coumarin-Phalloidin, CY3.1 8, CY5.1 8, CY7, Dans (1-Dimethyl Amino Naphaline 5 Sulphonic Acid), Dansa (Diamino Naphtyl Sulphonic Acid), Dansyl NH-CH3, Diamino Phenyl Oxydiazole (DAO), Dimethylamino-5-Sulphonic acid, Dipyrrometheneboron Difluoride, Diphenyl Brilliant Flavine 7GFF, Dopamine, Erythrosin ITC, Euchrysin, FIF (Formaldehyde Induced Fluorescence), Flazo Orange, Fluo 3, Fluorescamine, Fura-2, Genacryl Brilliant Red B, Genacryl Brilliant Yellow 10GF, Genacryl Pink 3G, Genacryl Yellow 5GF, Gloxalic Acid, Granular Blue, Haematoporphyrin, Indo-1, Intrawhite Cf Liquid, Leucophor PAF, Leucophor SF, Leucophor WS, Lissamine Rhodamine B200 (RD200), Lucifer Yellow CH, Lucifer Yellow VS, Magdala Red, Marina Blue, Maxilon Brilliant Flavin 10 GFF, Maxilon Brilliant Flavin 8 GFF, MPS (Methyl Green Pyronine Stilbene), Mithramycin, NBD Amine, Nitrobenzoxadidole, Noradrenaline, Nuclear Fast Red, Nuclear Yellow, Nylosan Brilliant Flavin E8G, Oxadiazole, Pacific Blue, Pararosaniline (Feulgen), Phorwite AR Solution, Phorwite BKL, Phorwite Rev, Phorwite RPA, Phosphine 3R, Phthalocyanine, Phycoerythrin R, Polyazaindacene Pontochrome Blue Black, Porphyrin, Primuline, Procion Yellow, Pyronine, Pyronine B, Pyrozal Brilliant Flavin 7GF, Quinacrine Mustard, Rhodamine 123, Rhodamine 5 GLD, Rhodamine 6G, Rhodamine B, Rhodamine B 200, Rhodamine B Extra, Rhodamine BB, Rhodamine BG, Rhodamine WT, Serotonin, Sevron Brilliant Red 2B, Sevron Brilliant Red 4G, Sevron Brilliant Red B, Sevron Orange, Sevron Yellow L, SITS (Primuline), SITS (Stilbene Isothiosulphonic acid), Stilbene, Snarf 1, sulpho Rhodamine B Can C, Sulpho Rhodamine G Extra, Tetracycline, Thiazine Red R, Thioflavin S, Thioflavin TCN, Thioflavin 5, Thiolyte, Thiozol Orange, Tinopol CBS, True Blue, Ultralite, Uranine B, Uvitex SFC, Xylene Orange, and XRITC.

Preferred fluorescent labels are fluorescein (5-carboxyfluorescein-N-hydroxysuccinimide ester), rhodamine (5,6-tetramethyl rhodamine), and the cyanine dyes Cy3, Cy3.5, Cy5, Cy5.5 and Cy7. The absorption and emission maxima, respectively, for these fluors are: FITC (490 nm; 520 nm), Cy3 (554 nm; 568 nm), Cy3.5 (581 nm; 588 nm), Cy5 (652 nm: 672 nm), Cy5.5 (682 nm; 703 nm) and Cy7 (755 nm; 778 nm), thus allowing their simultaneous detection. Other examples of fluorescein dyes include 6-carboxyfluorescein (6-FAM), 2',4',1,4,-tetrachlorofluorescein (TET), 2',4',5',7',1,4-hexachlorofluorescein (HEX), 2',7'-dimethoxy-4', 5'-dichloro-6-carboxyrhodamine (JOE), 2'-chloro-5'-fluoro-7',8-fused phenyl-1,4-dichloro-6-carboxyfluorescein (NED), and 2'-chloro-7'-phenyl-1,4-dichloro-6-carboxyfluorescein (VIC). Fluorescent labels can be obtained from a variety of commercial sources, including Amersham Pharmacia Biotech, Piscataway, NJ; Molecular Probes, Eugene, OR; and Research Organics, Cleveland, Ohio.

A useful form of primary labeling is the use of fluorescent change primers during amplification. Fluorescent change primers exhibit a change in fluorescence intensity or wavelength based on a change in the form or conformation of the primer and the amplified nucleic acid. Stem quenched primers are primers that when not hybridized to a complementary sequence form a stem structure (either an intramolecular stem structure or an intermolecular stem structure) that brings a fluorescent label and a quenching moiety into proximity such that fluorescence from the label is quenched. When the primer binds to a complementary sequence, the stem is disrupted, the quenching moiety is no longer in proximity to the fluorescent label and fluorescence increases. In the disclosed method, stem quenched primers can be used as primers for nucleic acid synthesis and thus become incorporated into the synthesized or amplified nucleic acid. Examples of stem quenched primers are peptide nucleic acid quenched primers and hairpin quenched primers.

Peptide nucleic acid quenched primers are primers associated with a peptide nucleic acid quencher or a peptide nucleic acid fluor to form a stem structure. The primer contains a fluorescent label or a quenching moiety and is associated with either a peptide nucleic acid quencher or a peptide nucleic acid fluor, respectively. This puts the fluorescent label in proximity to the quenching moiety. When the primer is replicated, the peptide nucleic acid is displaced, thus allowing the fluorescent label to produce a fluorescent signal.

Hairpin quenched primers are primers that when not hybridized to a complementary sequence form a hairpin structure (and, typically, a loop) that brings a fluorescent label and a quenching moiety into proximity such that fluorescence from the label is quenched. When the primer binds to a complementary sequence, the stem is disrupted, the quenching moiety is no longer in proximity to the fluorescent label and fluorescence increases. Hairpin quenched primers are typically used as primers for nucleic acid synthesis and thus become incorporated into the synthesized or amplified nucleic acid. Examples of hairpin quenched primers are Amplifluor primers and scorpion primers.

Cleavage activated primers are primers where fluorescence is increased by cleavage of the primer. Generally, cleavage activated primers are incorporated into replicated strands and are then subsequently cleaved. Cleavage activated primers can include a fluorescent label and a quenching moiety in proximity such that fluorescence from the label is quenched. When the primer is clipped or digested (typically by the 5'-3' exonuclease activity of a polymerase during amplification), the quenching moiety is no longer in proximity to the fluorescent label and fluorescence increases. Little et al., Clin. Chem. 45:777-784 (1999), describe the use of cleavage activated primers. Use of cleavage activated primers is not preferred in the disclosed method.

### ii. Secondary Labeling with Detection Probes

Secondary labeling consists of using suitable molecular probes, referred to as detection probes, to detect the amplified nucleic acids. For example, a primer may be designed to contain, in its non-complementary portion, a known arbitrary sequence, referred to as a detection tag. A secondary hybridization step can be used to bind detection probes to these detection tags. The detection probes may be labeled as described above with, for example, an enzyme, fluorescent moieties, or radioactive isotopes. By using three detection tags per primer, and four fluorescent moieties per each detection probe, one may obtain a total of twelve fluorescent signals for every replicated strand. Detection probes can interact by hybridization or annealing via normal Watson-Crick base-pairing (or related alternatives) or can interact with double-stranded targets to form a triple helix. Such triplex-forming detection probes can be used in the same manner as other detection probes, such as in the form of fluorescent change probes.

A useful form of secondary labeling is the use of fluorescent change probes and primers in or following amplification. Hairpin quenched probes are probes that when not bound to a target sequence form a hairpin structure (and, typically, a loop) that brings a fluorescent label and a quenching moiety into proximity such that fluorescence from the label is quenched. When the probe binds to a target sequence, the stem is disrupted, the quenching moiety is no longer in proximity to the fluorescent label and fluorescence increases. Examples of hairpin quenched probes are molecular beacons, fluorescent triplex oligos, and QPNA probes.

Cleavage activated probes are probes where fluorescence is increased by cleavage of the probe. Cleavage activated probes can include a fluorescent label and a quenching moiety in proximity such that fluorescence from the label is quenched. When the probe is clipped or digested (typically by the 5'-3' exonuclease activity of a polymerase during or following amplification), the quenching moiety is no longer in proximity to the fluorescent label and fluorescence increases. TaqMan probes are an example of cleavage activated probes.

Cleavage quenched probes are probes where fluorescence is decreased or altered by cleavage of the probe. Cleavage quenched probes can include an acceptor fluorescent label and a donor moiety such that, when the acceptor and donor are in proximity, fluorescence resonance energy transfer from the donor to the acceptor causes the acceptor to fluoresce. The probes are thus fluorescent, for example, when hybridized to a target sequence. When the probe is clipped or digested (typically by the 5'-3' exonuclease activity of a polymerase during or after amplification), the donor moiety is no longer in proximity to the acceptor fluorescent label and fluorescence from the acceptor decreases. If the donor moiety is itself a fluorescent label, it can release energy as fluorescence (typically at a different wavelength than the fluorescence of the acceptor) when not in proximity to an acceptor. The overall effect would then be a reduction of acceptor fluorescence and an increase in donor fluorescence. Donor fluorescence in the case of cleavage quenched probes is equivalent to fluorescence generated by cleavage activated probes with the acceptor being the quenching moiety and the donor being the fluorescent label. Cleavable FRET (fluorescence resonance energy transfer) probes are an example of cleavage quenched probes.

Fluorescent activated probes are probes or pairs of probes where fluorescence is increased or altered by hybridization of the probe to a target sequence. Fluorescent activated probes can include an acceptor fluorescent label and a donor moiety such that, when the acceptor and donor are in proximity (when the probes are hybridized to a target sequence), fluorescence resonance energy transfer from the donor to the acceptor causes the acceptor to fluoresce. Fluorescent activated probes are typically pairs of probes designed to hybridize to adjacent sequences such that the acceptor and donor are brought into proximity. Fluorescent activated probes can also be single probes containing both a donor and acceptor where, when the probe is not hybridized to a target sequence, the donor and acceptor are not in proximity but where the donor and acceptor are brought into proximity when the probe hybridized to a target sequence. This can be accomplished, for example, by placing the donor and acceptor on opposite ends a the probe and placing target complement sequences at each end of the probe where the target complement sequences are complementary to adjacent sequences in a target sequence. If the donor moiety of a fluorescent activated probe is itself a fluorescent label, it can release energy as fluorescence (typically at a different wavelength than the fluorescence of the acceptor) when not in proximity to an acceptor (that is, when the probes are not hybridized to the target sequence). When the probes hybridize to a target sequence, the overall effect would then be a reduction of donor fluorescence and an increase in acceptor fluorescence. FRET probes are an example of fluorescent activated probes. Stem quenched primers (such as peptide nucleic acid quenched primers and hairpin quenched primers) can be used as secondary labels.

### iii. Multiplexing and Hybridization Array Detection

Detection of amplified nucleic acids can be multiplexed by using sets of different primers, each set designed for amplifying different target sequences. Only those primers that are able to find their targets will give rise to amplified products. There are two alternatives for capturing a given amplified nucleic acid to a fixed position in a solid-state detector. One is to include within the non-complementary portion of the primers a unique address tag sequence for each unique set of primers. Nucleic acid amplified using a given set of primers will then contain sequences corresponding to a specific address tag sequence. A second and preferred alternative is to use a sequence present in the target sequence as an address tag. The disclosed method can be easily multiplexed by, for example, using sets of different detection probes directed to different target sequences. Use of different fluorescent labels with different detection probes allows specific detection of different target sequences.

### 2. Combinatorial Multicolor Coding

One form of multiplex detection involves the use of a combination of labels that either fluoresce at different wavelengths or are colored differently. One of the advantages of fluorescence for the detection of hybridization probes is that several targets can be visualized simultaneously in the same sample. Using a combinatorial strategy, many more targets can be discriminated than the number of spectrally resolvable fluorophores. Combinatorial labeling provides the simplest way to label probes in a multiplex fashion since a probe fluor is either completely absent (-) or present in unit amounts (+); image analysis is thus more amenable to automation, and a number of experimental artifacts, such as differential photobleaching of the fluors and the effects of changing excitation source power spectrum, are avoided. Combinatorial labeling can be used with fluorescent change probes and primers.

The combinations of labels establish a code for identifying different detection probes and, by extension, different target molecules to which those detection probes are associated with. This labeling scheme is referred to as Combinatorial Multicolor Coding (CMC). Such coding is described by Speicher et al., Nature Genetics 12:368-375 (1996). Use of CMC is described in U.S. Patent No. 6,143,495. Any number of labels, which when combined can be separately detected, can be used for combinatorial multicolor coding. It is preferred that 2, 3, 4, 5, or 6 labels be used in combination. It is most preferred that 6 labels be used. The number of labels used establishes the number of unique label combinations that can be formed according to the formula 2^{N}-1, where N is the number of labels. According to this formula, 2 labels forms three label combinations, 3 labels forms seven label combinations, 4 labels forms 15 label combinations, 5 labels form 31 label combinations, and 6 labels forms 63 label combinations.

For combinatorial multicolor coding, a group of different detection probes are used as a set. Each type of detection probe in the set is labeled with a specific and unique combination of fluorescent labels. For those detection probes assigned multiple labels, the labeling can be accomplished by labeling each detection probe molecule with all of the required labels. Alternatively; pools of detection probes of a given type can each be labeled with one of the required labels. By combining the pools, the detection probes will, as a group, contain the combination of labels required for that type of detection probe. Where each detection probe is labeled with a single label, label combinations can also be generated by using primers with coded combinations of detection tags complementary to the different detection probes. In this scheme, the primers will contain a combination of detection tags representing the combination of labels required for a specific label code. Further illustrations are described in U.S. Patent No. 6,143,495. Use of pools of detection probes each probe with a single label is preferred when fluorescent change probes are used.

Speicher *et al.* describes a set of fluors and corresponding optical filters spaced across the spectral interval 350-770 nm that give a high degree of discrimination between all possible fluor pairs. This fluor set, which is preferred for combinatorial multicolor coding, consists of 4'-6-diamidino-2-phenylinodole (DAPI), fluorescein (FITC), and the cyanine dyes Cy3, Cy3.5, Cy5, Cy5.5 and Cy7. Any subset of this preferred set can also be used where fewer combinations are required. The absorption and emission maxima, respectively, for these fluors are: DAPI (350 nm; 456 nm), FITC (490 nm; 520 nm), Cy3 (554 nm; 568 nm), Cy3.5 (581 nm; 588 nm), Cy5 (652 nm; 672 nm), Cy5.5 (682 nm; 703 nm) and Cy7 (755 nm; 778 nm). The excitation and emission spectra, extinction coefficients and quantum yield of these fluors are described by Ernst et al., Cytometry 10:3-10 (1989), Mujumdar et al., Cytometry 10:11-19 (1989), Yu, Nucleic Acids Res. 22:3226-3232 (1994), and Waggoner, Meth. Enzymology 246:362-373 (1995). These fluors can all be excited with a 75W Xenon arc.

To attain selectivity, filters with bandwidths in the range of 5 to 16 nm are preferred. To increase signal discrimination, the fluors can be both excited and detected at wavelengths far from their spectral maxima. Emission bandwidths can be made as wide as possible. For low-noise detectors, such as cooled CCD cameras, restricting the excitation bandwidth has little effect on attainable signal to noise ratios. A list of preferred filters for use with the preferred fluor set is listed in Table 1 of Speicher *et al.* It is important to prevent infra-red light emitted by the arc lamp from reaching the detector; CCD chips are extremely sensitive in this region. For this purpose, appropriate IR blocking filters can be inserted in the image path immediately in front of the CCD window to minimize loss of image quality. Image analysis software can then be used to count and analyze the spectral signatures of fluorescent dots.

### i. Enzyme-linked Detection

Amplified nucleic acid labeled by incorporation of labeled nucleotides can be detected with established enzyme-linked detection systems. For example, amplified nucleic acid labeled by incorporation of biotin using biotin-16-UTP (Roche Molecular Biochemicals) can be detected as follows. The nucleic acid is immobilized on a solid glass surface by hybridization with a complementary DNA oligonucleotide (address probe) complementary to the target sequence (or its complement) present in the amplified nucleic acid. After hybridization, the glass slide is washed and contacted with alkaline phosphatase-streptavidin conjugate (Tropix, Inc., Bedford, MA). This enzyme-streptavidin conjugate binds to the biotin moieties on the amplified nucleic acid. The slide is again washed to remove excess enzyme conjugate and the chemiluminescent substrate CSPD (Tropix, Inc.) is added and covered with a glass cover slip. The slide can then be imaged in a Biorad Fluorimager.

### 3. Using Products of Circular Genome Amplification

The nucleic acids produced using the disclosed method can be used for any purpose. For example, the amplified nucleic acids can be analyzed (such as by sequencing, any technique using hybridization, e.g., of probe or primers) to determine characteristics of the amplified sequences or the presence or absence or certain sequences. The amplified nucleic acids can also be used as reagents for assays or other methods. For example, nucleic acids produced in the disclosed method can be coupled or adhered to a solid-state substrate. The resulting immobilized nucleic acids can be used as probes or indexes of sequences in a sample. Nucleic acids produced in the disclosed method can be coupled or adhered to a solid-state substrate in any suitable way. For example, nucleic acids generated by multiple strand displacement can be attached by adding modified nucleotides to the 3' ends of nucleic acids produced by strand displacement replication using terminal deoxynucleotidyl transferase, and reacting the modified nucleotides with a solid-state substrate or support thereby attaching the nucleic acids to the solid-state substrate or support.

Nucleic acids produced in the disclosed method also can be used as probes or hybridization partners. For example, sequences of interest can be amplified in the disclosed method and provide a ready source of probes. The replicated strands (produced in the disclosed method) can be cleaved prior to use as hybridization probes. For example, the replicated strands can be cleaved with DNAse I. The hybridization probes can be labeled as described elsewhere herein with respect to labeling of nucleic acids produce in the disclosed method.

Nucleic acids produced in the disclosed method also can be used for subtractive hybridization to identify sequences that are present in only one of a pair or set of samples. For example, amplified cDNA from different samples can be annealed and the resulting double-stranded material can be separated from single-stranded material. Unhybridized sequences would be indicative of sequences expressed in one of the samples but not others.

### Specific Embodiments

Disclosed is a method of amplifying a circular genome, as defined in claim 1, the method comprising, bringing into contact a set of primers, DNA polymerase, and a genomic nucleic acid sample, wherein the genomic nucleic acid sample comprises a circular genome, and incubating the genomic nucleic acid sample under conditions that promote replication of the circular genome in the genomic nucleic acid sample, wherein replication of the circular genome proceeds by rolling circle replication, wherein the conditions that promote replication of the circular genome do not involve thermal cycling, wherein the genomic nucleic acid sample further comprises non-target nucleic acids, wherein the circular genome is amplified at least 10 fold compared to the non-target nucleic acids.

The non-target nucleic acids can comprise one or more non-target genomes, wherein the circular genome is amplified at least 10 fold compared to the non-target genomes. The circular genome can be amplified at least 50 fold, 100 fold, 200 fold, 500 fold, 1000 fold, 2000 fold, or 5000 fold compared to the non-target genomes. The non-target genome can be a bacterial genome, viral genome, microbial genome, pathogen genome, eukaryotic genome, plant genome, animal genome, vertebrate genome, fish genome, avian genome, mammalian genome, rodent genome, murine genome, human genome, host genome, a non-target circular genome, or a combination. The circular genome can be an organelle genome, a mitochondrial genome, a chloroplast genome, a plastid genome, a bacterial plasmid genome, a viral genome, a bacterial genome, a microbial genome, a pathogen genome, or a combination. The circular genome can be a naturally occurring genome. The circular genome can not be artificially modified. The circular genome can not be an artificial nucleic acid. The circular genome can be double-stranded or single-stranded. The circular genome can have a length of from about 3000 to about 300000 nucleotides, about 4000 to about 260000 nucleotides, about 5000 to about 150000 nucleotides, or about 5500 to about 40000 nucleotides.

The primers can each comprise a specific nucleotide sequence. The primers can each have a specific nucleotide sequence. The primers can specifically hybridize to a nucleotide sequence in the circular genome under conditions that promote replication of the circular genome. The primers can each separately have a length 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, or 30 nucleotides.

The primers can each separately have a length less than 6 nucleotides, less than 7 nucleotides, less than 8 nucleotides, less than 9 nucleotides, less than 10 nucleotides, less than 11 nucleotides, less than 12 nucleotides, less than 13 nucleotides, less than 14 nucleotides, less than 15 nucleotides, less than 16 nucleotides, less than 17 nucleotides, less than 18 nucleotides, less than 19 nucleotides, less than 20 nucleotides, less than 21 nucleotides, less than 22 nucleotides, less than 23 nucleotides, less than 24 nucleotides, less than 25 nucleotides, less than 26 nucleotides, less than 27 nucleotides, less than 28 nucleotides, less than 29 nucleotides, less than 30 nucleotides, or less than 31 nucleotides.

The set of primers can comprise 2 primers, 3 primers, 4 primers, 5 primers, 6 primers, 7 primers, 8 primers, 9 primers, 10 primers, 11 primers, 12 primers, 13 primers, 14 primers, 15 primers, 16 primers, 17 primers, 18 primers, 19 primers, 20 primers, 21 primers, 22 primers, 23 primers, 24 primers, 25 primers, 26 primers, 27 primers, 28 primers, 29 primers, 30 primers, 31 primers, 32 primers, 33 primers, 34 primers, 35 primers, 36 primers, 37 primers, 38 primers, 39 primers, 40 primers, 41 primers, 42 primers, 43 primers, 44 primers, 45 primers, 46 primers, 47 primers, 48 primers, 49 primers, 50 primers, 51 primers, 52 primers, 53 primers, 54 primers, 55 primers, 56 primers, 57 primers, 58 primers, 59 primers, 60 primers, 61 primers, 62 primers, 63 primers, 75 primers, 100 primers, 150 primers, 200 primers, 300 primers, 400 primers, wherein each primer in the set has a different specific nucleotide sequence.

The primers can each have a nucleotide sequence complementary to a nucleotide sequence in the circular genome, wherein the distance between consecutive primers hybridized to the same strand of the circular genome averages from about 200 to about 20000 nucleotides, about 200 to about 6000 nucleotides, about 300 to about 5000 nucleotides, or about 400 to about 4000 nucleotides. The primers can each have a nucleotide sequence complementary to a nucleotide sequence in the circular genome, wherein the distance between consecutive primers hybridized to the same strand of the circular genome are from about 200 to about 20000 nucleotides, about 200 to about 6000 nucleotides, about 300 to about 5000 nucleotides, or about 400 to about 4000 nucleotides. The circular genome can be double-stranded, wherein one or more of the primers have a nucleotide sequence complementary to one of the strands of the circular genome and one or more of the primers have a nucleotide sequence complementary to the other strand of the circular genome, wherein all of the primers have a nucleotide sequence complementary to one of the strands of the circular genome, or all of the primers have a nucleotide sequence complementary to the other strand of the circular genome.

The circular genome can be single-stranded, wherein one or more of the primers have a nucleotide sequence complementary to the circular genome and one or more of the primers have a nucleotide sequence that matches a sequence of the circular genome, wherein rolling circle replication results in the formation of tandem sequence DNA, wherein the one or more of the primers that have a nucleotide sequence that matches a sequence of the circular genome prime strand displacement replication of the tandem sequence DNA, wherein replication of the tandem sequence DNA results in formation of secondary tandem sequence DNA.

The primers can each separately comprise deoxyribonucleotides, ribonucloetides, modified nucleotides, nucleotide analogs, labelled nucleotides, oligomer analogs, or a combination.

The genomic nucleic acid sample can be a blood sample, a urine sample, a semen sample, a lymphatic fluid sample, a cerebrospinal fluid sample, amniotic fluid sample, a biopsy sample, a needle aspiration biopsy sample, a cancer sample, a tumor sample, a tissue sample, a cell sample, a cell lysate sample, a crude cell lysate sample, a forensic sample, an archeological sample, an infection sample, a nosocomial infection sample, an environmental sample, or a combination thereof.

The conditions that promote replication of the circular genome can be substantially isothermic.

Also disclosed is a method of identifying a set of primers for differential amplification of a circular genome, the method comprising selecting test primers for a test set of primers, wherein each primer can specifically hybridize to a nucleotide sequence in a circular genome, wherein the distance between consecutive primers hybridized to the same strand of the circular genome averages from about 200 to about 20000 nucleotides, about 200 to about 6000 nucleotides, bringing into contact the test set of primers, DNA polymerase, and a genomic nucleic acid sample, wherein the test genomic nucleic acid sample comprises the circular genome and non-target nucleic acids, incubating the genomic nucleic acid sample under conditions that promote replication of the circular genome in the genomic nucleic acid sample, wherein replication of the circular genome proceeds by rolling circle replication, wherein the conditions that promote replication of the circular genome do not involve thermal cycling, and determining the relative amplification of the circular genome and the non-target nucleic acids, wherein the test set of primers are identified if the circular genome is amplified at least 10 fold compared to the non-target nucleic acids.

### Example

### A. Differential Amplification of Mitochondrial Genome

This example describes amplification of mitochondrial DNA according the disclosed method. A nucleic acid preparation isolated by the QIAamp procedure (QIAGEN) from human blood contains both mitochondrial DNA in modest amounts and a large amount of nuclear DNA. Primers were selected according to the principles disclosed herein that bind specifically to mitochondrial DNA. Amplification using primers10 nucleotides in length were compared with amplification using primers 14 nucleotides in length. The primer sequences are shown in Table 1. The distance between consecutive primers on each strand of the mitochondrial DNA was about 4000 nucleotides.

**Table 1. Mitochondrial specific primers:**

| 10 nucleotide long primers: | |
|---|---|
| mt1s2 | CCCCATTC*C*A |
| mt2as2 | CAATTGAG*T*A |
| mt3s2 | AATCCTTC*T*A |
| mt4as2 | CGGTCTGT*T*A |
| mt5s2 | GCCACAAC*T*A |
| mt6as2 | TATGAGAG*T*A |
| mt7s2 | CAAACATC*T*A |
| mt8as2 | CGTGGTTG*T*A) |
| | |

| 14 nucleotide long primers: | |
|---|---|
| mt1s3 | GCATCCCCATTC*C*A |
| mt2as3 | TTCCCAATTGAG*T*A |
| mt3s3 | CCATAATCCTTC*T*A |
| mt4as3 | GTTGCGGTCTGT*T*A |
| mt5s3 | TATTGCCACAAC*T*A |
| mt6as3 | GGGTTATGAGAG*T*A |
| mt7s3 | GACCCAAACATC*T*A |
| mt8as3 | TGGTCGTGGTTG*T*A |

Asterisks indicate Exonuclease resistant Phosphorothioate Modification.

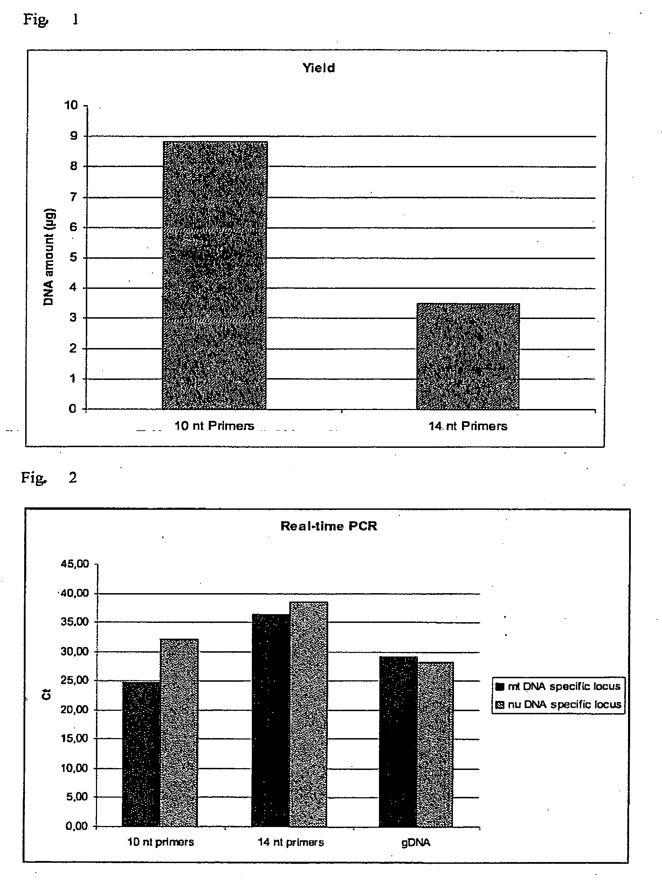

10 ng of DNA (containing nuclear and mitochondrial DNA) was added to an REPLI-g buffer (QIAGEN) with or without primers. For reactions with primers, either the eight 10 nucleotide primers or the eight 14 nucleotide primers were added. The mixture was heated to 70°C for 5 min. After heating the reaction was cooled down on ice and Phi29 DNA Polymerase was added. The amplification was performed for eight hours at 30°C. The resulting nucleic acid was quantified by PicoGreen Assay (Livitrogen). The results are shown in Figure 1. The 10 nucleotide primers resulting in significantly greater yield than the 14 nucleotide primers. This was not expected and shows that shorter primers can have a thermodynamic advantage over longer primers in rolling circle amplification of circular genomes. The shorter primers resulted in not only a higher specificity (as shown by lower CT values, see below) but also and unexpectedly in a better amplification rate (Figure 1).

The differential amplification of the mitochondrial DNA over nuclear DNA was determined by real-time PCR using 10 ng of amplified DNA from the amplification reaction or 10 ng of the unamplified source nucleic acid. Single sequences in the mitochondrial DNA and in the nuclear DNA were used for this assessment.

### Primers for real-time PCR System for Nuclear DNA

| | |
|---|---|
| 699s | TGCTCCCTGTCCCATCTG |
| 699as | AGACAGTATGCCTTTATTTCACCC |

### Primers for real-time PCR System for Mitochondrial DNA

| | |
|---|---|
| mt9s / 665 | CTCTTGCTCAGCCTATATAC |
| mt10as/665 | GTAGAAAATGTAGCCCATTAC |

The results are shown in Figure 2. The Ct values between nuclear and mitochondrial specific markers are very similar if using gDNA (=nucleic acid sample) that contain both nuclear DNA and mitochondrial DNA. The Ct value of the mitochondrial marker is decreased by - 4 cycles after having performed the mitochondrial specific RCA using 10 nt long mitochondrial DNA specific primers. In contrast, the Ct value of the nuclear marker is increased by ~ 4 cycles after the mitochondrial specific RCA. In contrast, the results obtained with 14 nt long primers did not result in a highly specific amplification of mitochondrial DNA: Both, the Ct values of nuclear and mitochondrial specific markers increased. This indicated that an amplification of a non-specific DNA has occured. It has been described in the art that non-specific DNA can be generated in the presence of primers by forming large artificial DNA molecules starting from intermolecular primer aggregates. In addition, a lower yield was observed with 14 nt Primers as compared to 10 nt long primers (see Figure 1). This indicates that the DNA polymerase preferres to build an elongation complex with the shorter primers rather than with the longer primers.

## Claims

1. A method of amplifying a circular genome, the method comprising,
bringing into contact a set of primers, DNA polymerase, and a genomic nucleic acid sample, wherein the genomic nucleic acid sample comprises a circular genome, and
incubating the genomic nucleic acid sample under conditions that promote replication of the circular genome in the genomic nucleic acid sample, wherein replication of the circular genome proceeds by rolling circle replication, wherein the conditions that promote replication of the circular genome do not involve thermal cycling, wherein the genomic nucleic acid sample further comprises non-target nucleic acids, wherein the circular genome is amplified at least 10 fold compared to the non-target nucleic acids wherein the polymerase is a Phi29 DNA polymerase, wherein the conditions that involve replication involve use of a Tris buffer with a pH of 8.5 to 9 and wherein the conditions that promote replication of the circular genome are substantially isothermic.

2. Method according to claim 1, wherein the buffer has a pH of 8.5.

3. Method according to claim 1 or 2, wherein the primers comprise or have a specific nucleotide sequence.

4. The method of claims 1 to 3, wherein the non-target nucleic acids comprise one or more non-target genomes, wherein the circular genome is amplified at least 10 fold compared to the non-target genomes.

5. The method of claims 1 to 4, wherein the circular genome is amplified at least 50 fold, 100 fold, 200 fold, 500 fold, 1000 fold, 2000 fold, or 5000 fold compared to the non-target genomes.

6. The method of claims 1 or 5, wherein the non-target genome is a bacterial genome, viral genome, microbial genome, pathogen genome, eukaryotic genome, plant genome, animal genome, vertebrate genome, fish genome, avian genome, mammalian genome, rodent genome, murine genome, human genome, host genome, a non-target circular genome, or a combination.

7. The method of any of claims 1 to 6, wherein the circular genome is an organelle genome, a mitochondrial genome, a chloroplast genome, a plastid genome, a bacterial plasmid genome, a viral genome, a bacterial genome, a microbial genome, a pathogen genome, or a combination.

8. The method of any of claims 1 to 7, wherein the circular genome is double-stranded or single-stranded.

9. The method of any of claims 1 to 8, wherein the circular genome has a length of from about 3000 to about 300000 nucleotides, about 4000 to about 260000 nucleotides, about 5000 to about 150000 nucleotides, or about 5500 to about 40000 nucleotides.

10. The method of any of claims 1 to 9, wherein the primers each separately have a length 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, or 30 nucleotides.

11. The method of any of claims 1 to 10, wherein the primers each separately have a length less than 6 nucleotides, less than 7 nucleotides, less than 8 nucleotides, less than 9 nucleotides, less than 10 nucleotides, less than 11 nucleotides, less than 12 nucleotides, less than 13 nucleotides, less than 14 nucleotides, less than 15 nucleotides, less than 16 nucleotides, less than 17 nucleotides, less than 18 nucleotides, less than 19 nucleotides, less than 20 nucleotides, less than 21 nucleotides, less than 22 nucleotides, less than 23 nucleotides, less than 24 nucleotides, less than 25 nucleotides, less than 26 nucleotides, less than 27 nucleotides, less than 28 nucleotides, less than 29 nucleotides, less than 30 nucleotides, or less than 31 nucleotides.

12. The method of any of claims 1 11, wherein the set of primers comprises 2 primers, 3 primers, 4 primers, 5 primers, 6 primers, 7 primers, 8 primers, 9 primers, 10 primers, 11 primers, 12 primers, 13 primers, 14 primers, 15 primers, 16 primers, 17 primers, 18 primers, 19 primers, 20 primers, 21 primers, 22 primers, 23 primers, 24 primers, 25 primers, 26 primers, 27 primers, 28 primers, 29 primers, 30 primers, 31 primers, 32 primers, 33 primers, 34 primers, 35 primers, 36 primers, 37 primers, 38 primers, 39 primers, 40 primers, 41 primers, 42 primers, 43 primers, 44 primers, 45 primers, 46 primers, 47 primers, 48 primers, 49 primers, 50 primers, 51 primers, 52 primers, 53 primers, 54 primers, 55 primers, 56 primers, 57 primers, 58 primers, 59 primers, 60 primers, 61 primers, 62 primers, 63 primers, 75 primers, 100 primers, 150 primers, 200 primers, 300 primers, 400 primers, wherein each primer in the set has a different specific nucleotide sequence.

13. The method of any of claims 1 to 12, wherein the primers each have a nucleotide sequence complementary to a nucleotide sequence in the circular genome, wherein the distance between consecutive primers hybridized to the same strand of the circular genome averages from about 200 to about 20000 nucleotides, about 200 to about 6000 nucleotides, about 300 to about 5000 nucleotides, or about 400 to about 4000 nucleotides.

14. The method of any of claims 1 to 13, wherein the primers each have a nucleotide sequence complementary to a nucleotide sequence in the circular genome, wherein the distance between consecutive primers hybridized to the same strand of the circular genome are from about 200 to about 20000 nucleotides, about 200 to about 6000 nucleotides, about 300 to about 5000 nucleotides, or about 400 to about 4000 nucleotides.

15. The method of any of claims 1 to 14, wherein the circular genome is double-stranded, wherein one or more of the primers have a nucleotide sequence complementary to one of the strands of the circular genome and one or more of the primers have a nucleotide sequence complementary to the other strand of the circular genome, wherein all of the primers have a nucleotide sequence complementary to one of the strands of the circular genome, or all of the primers have a nucleotide sequence complementary to the other strand of the circular genome.

16. The method of any of claims 1 to 15, wherein the circular genome is single-stranded, wherein one or more of the primers have a nucleotide sequence complementary to the circular genome and one or more of the primers have a nucleotide sequence that matches a sequence of the circular genome, wherein rolling circle replication results in the formation of tandem sequence DNA, wherein the one or more of the primers that have a nucleotide sequence that matches a sequence of the circular genome prime strand displacement replication of the tandem sequence DNA, wherein replication of the tandem sequence DNA results in formation of secondary tandem sequence DNA.

17. The method of any of claims 1 to 16 wherein the primers each separately comprise deoxyribonucleotides, ribonucloetides, modified nucleotides, nucleotide analogs, labelled nucleotides, oligomer analogs, or a combination.

18. The method of any of claims 1 to 17, wherein the genomic nucleic acid sample is a blood sample, a urine sample, a semen sample, a lymphatic fluid sample, a cerebrospinal fluid sample, amniotic fluid sample, a biopsy sample, a needle aspiration biopsy sample, a cancer sample, a tumor sample, a tissue sample, a cell sample, a cell lysate sample, a crude cell lysate sample, a forensic sample, an archeological sample, an infection sample, a nosocomial infection sample, an environmental sample, or a combination thereof.

## Patentansprüche

1. Verfahren zur Amplifikation eines zirkulären Genoms, umfassend
Inkontaktbringen eines Primer-Satzes, einer DNA Polymerase und einer genomischen Nukleinsäure-Probe, wobei die genomische Nukleinsäure-Probe ein zirkuläres Genom umfasst, und
Inkubieren der genomischen Nukleinsäure-Probe unter Bedingungen, die die Replikation des zirkulären Genoms in der genomischen Nukleinsäure-Probe begünstigt, wobei die Replikation des zirkulären Genoms durch eine Rolling Circle Replikation verläuft, wobei die Bedingungen, die die Replikation des zirkulären Genoms begünstigen, keine thermale Amplifikation umfassen, wobei die genomische Nukleinsäure-Probe ferner Nicht-Zielnukleinsäuren umfasst, wobei das zirkuläre Genom wenigstens 10-fach amplifiziert wird verglichen mit der Nicht-Zielnukleinsäure, wobei die Polymerase eine Phi29 DNA Polymerase ist, wobei die Bedingungen, die die Replikation verursachen, die Verwendung eines Tris-Puffers mit einem pH von 8,5 bis 9 beinhalten und wobei die Bedingungen, die die Replikation des zirkulären Genoms begünstigen, im Wesentlichen isotherm sind.

2. Verfahren gemäß Anspruch 1, wobei der Puffer einen pH von 8,5 hat.

3. Verfahren gemäß Anspruch 1 oder 2, wobei die Primer eine spezifische Nukleotidsequenz umfassen oder haben.

4. Verfahren nach den Ansprüchen 1 bis 3, wobei die Nicht-Zielnukleinsäure einen oder mehrere Nicht-Zielgenome umfassen, wobei das zirkuläre Genom wenigstens 10-fach verglichen mit den Nicht-Zielgenomen amplifiziert wird.

5. Verfahren der Ansprüche 1 bis 4, wobei das zirkuläre Genom wenigstens 50-fach, 100-fach, 200-fach, 500-fach, 1000-fach, 2000-fach oder 5000-fach verglichen zu den Nicht-Zielgenomen amplifiziert wird.

6. Verfahren der Ansprüche 1 oder 5, wobei das Nicht-Zielgenom ein bakterielles Genom, virales Genom, mikrobielles Genom, Pathogengenom, eukaryotisches Genom, Pflanzengenom, Tiergenom, Wirbeltiergenom, Fischgenom, Vogelgenom, Säugetiergenom, Nagergenom, Mausgenom, Menschgenom, Wirtsgenom, ein nicht-Ziel-zirkuläres Genom oder eine Kombination ist.

7. Verfahren nach einem der Ansprüche 1 bis 6, wobei das zirkuläre Genom ein Organellengenom, ein mitochondriales Genom, ein Chloroplastengenom, ein Plastidgenom, ein bakterielles Plasmidgenom, ein virales Genom, ein bakterielles Genom, ein mikrobielles Genom, ein Pathogengenom oder ein Kombination ist.

8. Verfahren nach einem der Ansprüche 1 bis 7, wobei das zirkuläre Genom doppelsträngig oder einzelsträngig ist.

9. Verfahren nach einem der Ansprüche 1 bis 8, wobei das zirkuläre Genom eine Länge von etwa 3.000 bis etwa 300.000 Nukleotiden, etwa 4.000 bis etwa 260.000 Nukleotiden, etwa 5.000 bis etwa 150.000 Nukleotiden oder etwa 5.500 bis etwa 40.000 Nukleotiden hat.

10. Verfahren nach einem der Ansprüche 1 bis 9, wobei die Primer jeweils eine Länge von 5 Nukleotiden, 6 Nukleotiden, 7 Nukleotiden, 8 Nukleotiden, 9 Nukleotiden, 10 Nukleotiden, 11 Nukleotiden, 12 Nukleotiden, 13 Nukleotiden, 14 Nukleotiden, 15 Nukleotiden, 16 Nukleotiden, 17 Nukleotiden, 18 Nukleotiden, 19 Nukleotiden, 20 Nukleotiden, 21 Nukleotiden, 22 Nukleotiden, 23 Nukleotiden, 24 Nukleotiden, 25 Nukleotiden, 26 Nukleotiden, 27 Nukleotiden, 28 Nukleotiden, 29 Nukleotiden oder 30 Nukleotiden haben.

11. Verfahren nach einem der Ansprüche 1 bis 10, wobei die Primer jeweils eine Länge von weniger als 6 Nukleotide, weniger als 7 Nukleotide, weniger als 8 Nukleotide, weniger als 9 Nukleotide, weniger als 10 Nukleotide, weniger als 11 Nukleotide, weniger als 12 Nukleotide, weniger als 13 Nukleotide, weniger als 14 Nukleotide, weniger als 15 Nukleotide, weniger als 16 Nukleotide, weniger als 17 Nukleotide, weniger als 18 Nukleotide, weniger als 19 Nukleotide, weniger als 20 Nukleotide, weniger als 21 Nukleotide, weniger als 22 Nukleotide, weniger als 23 Nukleotide, weniger als 24 Nukleotide, weniger als 25 Nukleotide, weniger als 26 Nukleotide, weniger als 27 Nukleotide, weniger als 28 Nukleotide, weniger als 29 Nukleotide, weniger als 30 Nukleotide oder weniger als 31 Nukleotide haben.

12. Verfahren nach einem der Ansprüche 1 bis 11, wobei der Primersatz 2 Primer, 3 Primer, 4 Primer, 5 Primer, 6 Primer, 7 Primer, 8 Primer, 9 Primer, 10 Primer, 11 Primer, 12 Primer, 13 Primer, 14 Primer, 15 Primer, 16 Primer, 17 Primer, 18 Primer, 19 Primer, 20 Primer, 21 Primer, 22 Primer, 23 Primer, 24 Primer, 25 Primer, 26 Primer, 27 Primer, 28 Primer, 29 Primer, 30 Primer, 31 Primer, 32 Primer, 33 Primer, 34 Primer, 35 Primer, 36 Primer, 37 Primer, 38 Primer, 39 Primer, 40 Primer, 41 Primer, 42 Primer, 43 Primer, 44 Primer, 45 Primer, 46 Primer, 47 Primer, 48 Primer, 49 Primer, 50 Primer, 51 Primer, 52 Primer, 53 Primer, 54 Primer, 55 Primer, 56 Primer, 57 Primer, 58 Primer, 59 Primer, 60 Primer, 61 Primer, 62 Primer, 63 Primer, 75 Primer, 100 Primer, 150 Primer, 200 Primer, 300 Primer, 400 Primer umfasst, wobei jeder Primer in dem Satz eine andere spezifische Nukleotidsequenz hat.

13. Verfahren nach einem der Ansprüche 1 bis 12, wobei die Primer jeweils eine Nukleotidsequenz komplementär zu einer Nukleotidsequenz in dem zirkulären Genom haben, wobei der Abstand zwischen aufeinanderfolgenden Primern, die an demselben Strang des zirkulären Genoms hybridisieren, im Schnitt zwischen etwa 200 und etwa 20.000 Nukleotiden, etwa 200 bis etwa 6.000 Nukleotiden, etwa 300 bis etwa 5.000 Nukleotiden oder etwa 400 bis etwa 4.000 Nukleotiden ist.

14. Verfahren nach einem der Ansprüche 1 bis 13, wobei die Primer jeweils eine Nukleotidsequenz komplementär zu einer Nukleotidsequenz in dem zirkulären Genom haben, wobei der Abstand zwischen aufeinanderfolgenden Primern, die an demselben Strang des zirkulären Genoms hybridisieren, etwa 200 bis etwa 20.000 Nukleotiden, etwa 200 bis etwa 6.000 Nukleotiden, etwa 300 bis 5.000 Nukleotiden oder etwa 400 bis etwa 4.000 Nukleotiden ist.

15. Verfahren nach einem der Ansprüche 1 bis 14, wobei das zirkuläre Genom doppelsträngig ist, wobei einer oder mehrere der Primer eine Nukleotidsequenz komplementär zu einem der Stränge des zirkulären Genoms haben und einer oder mehrere der Primer eine Nukleotidsequenz komplementär zu dem anderen Strang des zirkulären Genoms haben, wobei alle der Primer eine Nukleotidsequenz komplementär zu einem der Stränge des zirkulären Genoms haben oder alle der Primer eine Nukleotidsequenz komplementär zu dem anderen Strang des zirkulären Genoms haben.

16. Verfahren nach einem der Ansprüche 1 bis 15, wobei das zirkuläre Genom einzelsträngig ist, wobei eine oder mehrere der Primer eine Nukleotidsequenz komplementär zu dem zirkulären Genom haben und einer oder mehrere der Primer eine Nukleotidsequenz haben, die einer Sequenz des zirkulären Genoms entspricht, wobei die Rolling Circle Replikation zu der Bildung einer Tandem-Sequenz-DNA führt, wobei der eine oder mehrere der Primer, die eine Nukleotidsequenz haben, die einer Sequenz des zirkulären Genoms entspricht, Strangverlagerungsreplikation der Tandem Sequenz DNA begünstigen, wobei die Replikation der Tandem-Sequenz-DNA zur Bildung einer DNA zur Bildung einer sekundären Tandem-Sequenz-DNA führt.

17. Verfahren nach einem der Ansprüche 1 bis 16, wobei die Primer jeweils Deoxyribonukleotide, Ribonukleotide, modifizierte Nukleotide, Nukleotid-Analoga, markierte Nukleotide, Oligomer-Analoga oder Kombination umfassen.

18. Verfahren nach einem der Anspräche 1 bis 17, wobei die genomische Nukleinsäure-Probe eine Blutprobe, eine Urinprobe, eine Samenprobe, eine lymphatische Flüssigkeitsprobe, eine Zerebrospinalflüssigkeitsprobe, amniotische Flüssigkeitsprobe, eine Biopsieprobe , eine Nadelaspirationsbiopsieprobe, eine Krebsprobe, eine Tumorprobe, eine Gewebeprobe, eine Zellprobe, eine Zelllysatprobe, eine Rohzelllysatprobe, eine forensische Probe, eine archäologische Probe, eine infektiöse Probe, eine nosokomiale infektiöse Probe, eine Umweltprobe oder einer Kombination davon ist.

## Revendications

1. Procédé d'amplification d'un génome circulaire, le procédé comprenant :
la mise en contact d'un ensemble d'amorces, d'une ADN polymérase et d'un échantillon d'acide nucléique génomique, dans lequel l'échantillon d'acide nucléique génomique comprend un génome circulaire, et
l'incubation de l'échantillon d'acide nucléique génomique dans des conditions favorisant la réplication du génome circulaire dans l'échantillon d'acide nucléique génomique, dans lequel la réplication du génome circulaire consiste en une réplication en cercle roulant, dans lequel les conditions favorisant la réplication du génome circulaire n'impliquent pas de cyclage thermique, dans lequel l'échantillon d'acide nucléique génomique comprend en outre des acides nucléiques non ciblés,
dans lequel le génome circulaire est amplifié au moins 10 fois par rapport aux acides nucléiques non ciblés, dans lequel la polymérase est une ADN polymérase Phi29,
dans lequel les conditions impliquant la réplication impliquent également l'utilisation d'une solution tampon Tris au pH allant de 8,5 à 9, et dans lequel les conditions favorisant la réplication du génome circulaire sont sensiblement isothermes.

2. Procédé selon la revendication 1, dans lequel la solution tampon a un pH de 8,5.

3. Procédé selon la revendication 1 ou 2, dans lequel les amorces comprennent ou ont une séquence nucléotidique spécifique.

4. Procédé selon les revendications 1 à 3, dans lequel les acides nucléiques non ciblés comprennent un ou plusieurs génomes non ciblés, dans lequel le génome circulaire est amplifié au moins 10 fois par rapport aux génomes non ciblés.

5. Procédé selon les revendications 1 à 4, dans lequel le génome circulaire est amplifié au moins 50 fois, 100 fois, 200 fois, 500 fois, 1 000 fois, 2 000 fois, ou 5 000 fois par rapport aux génomes non ciblés.

6. Procédé selon la revendication 1 ou 5, dans lequel le génome non ciblé est un génome bactérien, un génome viral, un génome microbien, un génome pathogène, un génome eucaryote, un génome de plante, un génome animal, un génome de vertébré, un génome de poisson, un génome aviaire, un génome de mammifère, un génome de rongeur, un génome murin, un génome humain, un génome hôte, un génome circulaire non ciblé, ou une combinaison.

7. Procédé selon l'une quelconque des revendications 1 à 6, dans lequel le génome circulaire est un génome d'organite, un génome mitochondrial, un génome de chloroplaste, un génome de plaste, un génome de plasmide bactérien, un génome viral, un génome bactérien, un génome microbien, un génome pathogène, ou une combinaison.

8. Procédé selon l'une quelconque des revendications 1 à 7, dans lequel le génome circulaire est un génome à double brin ou monobrin.

9. Procédé selon l'une quelconque des revendications 1 à 8, dans lequel le génome circulaire a une longueur allant d'environ 3 000 à environ 300 000 nucléotides, d'environ 4 000 à environ 260 000 nucléotides, d'environ 5 000 à environ 150 000 nucléotides, ou d'environ 5 500 à environ 40 000 nucléotides.

10. Procédé selon l'une quelconque des revendications 1 à 9, dans lequel les amorces ont chacune séparément une longueur correspondant à 5 nucléotides, 6 nucléotides, 7 nucléotides, 8 nucléotides, 9 nucléotides, 10 nucléotides, 11 nucléotides, 12 nucléotides, 13 nucléotides, 14 nucléotides, 15 nucléotides, 16 nucléotides, 17 nucléotides, 18 nucléotides, 19 nucléotides, 20 nucléotides, 21 nucléotides, 22 nucléotides, 23 nucléotides, 24 nucléotides, 25 nucléotides, 26 nucléotides, 27 nucléotides, 28 nucléotides, 29 nucléotides, ou 30 nucléotides.

11. Procédé selon l'une quelconque des revendications 1 à 10, dans lequel les amorces ont chacune séparément une longueur correspondant à moins de 6 nucléotides, moins de 7 nucléotides, moins de 8 nucléotides, moins de 9 nucléotides, moins de 10 nucléotides, moins de 11 nucléotides, moins de 12 nucléotides, moins de 13 nucléotides, moins de 14 nucléotides, moins de 15 nucléotides, moins de 16 nucléotides, moins de 17 nucléotides, moins de 18 nucléotides, moins de 19 nucléotides, moins de 20 nucléotides, moins de 21 nucléotides, moins de 22 nucléotides, moins de 23 nucléotides, moins de 24 nucléotides, moins de 25 nucléotides, moins de 26 nucléotides, moins de 27 nucléotides, moins de 28 nucléotides, moins de 29 nucléotides, moins de 30 nucléotides, ou moins de 31 nucléotides.

12. Procédé selon l'une quelconque des revendications 1 à 11, dans lequel l'ensemble d'amorces comprend 2 amorces, 3 amorces, 4 amorces, 5 amorces, 6 amorces, 7 amorces, 8 amorces, 9 amorces, 10 amorces, 11 amorces, 12 amorces, 13 amorces, 14 amorces, 15 amorces, 16 amorces, 17 amorces, 18 amorces, 19 amorces, 20 amorces, 21 amorces, 22 amorces, 23 amorces, 24 amorces, 25 amorces, 26 amorces, 27 amorces, 28 amorces, 29 amorces, 30 amorces, 31 amorces, 32 amorces, 33 amorces, 34 amorces, 35 amorces, 36 amorces, 37 amorces, 38 amorces, 39 amorces, 40 amorces, 41 amorces, 42 amorces, 43 amorces, 44 amorces, 45 amorces, 46 amorces, 47 amorces, 48 amorces, 49 amorces, 50 amorces, 51 amorces, 52 amorces, 53 amorces, 54 amorces, 55 amorces, 56 amorces, 57 amorces, 58 amorces, 59 amorces, 60 amorces, 61 amorces, 62 amorces, 63 amorces, 75 amorces, 100 amorces, 150 amorces, 200 amorces, 300 amorces, 400 amorces, dans lequel chaque amorce dans l'ensemble d'amorces a une séquence nucléotidique spécifique différente.

13. Procédé selon l'une quelconque des revendications 1 à 12, dans lequel les amorces ont chacune une séquence nucléotidique complémentaire d'une séquence nucléotidique dans le génome circulaire, dans lequel la distance entre deux amorces consécutives hybridées au même brin du génome circulaire est en moyenne d'environ 200 à environ 20 000 nucléotides, d'environ 200 à environ 6 000 nucléotides, d'environ 300 à environ 5 000 nucléotides, ou d'environ 400 à environ 4 000 nucléotides.

14. Procédé selon l'une quelconque des revendications 1 à 13, dans lequel les amorces ont chacune une séquence nucléotidique complémentaire d'une séquence nucléotidique dans le génome circulaire, dans lequel la distance entre deux amorces consécutives hybridées au même brin du génome circulaire est d'environ 200 à environ 20 000 nucléotides, d'environ 200 à environ 6 000 nucléotides, d'environ 300 à environ 5 000 nucléotides, ou d'environ 400 à environ 4 000 nucléotides.

15. Procédé selon l'une quelconque des revendications 1 à 14, dans lequel le génome circulaire est un génome à double brin, dans lequel une ou plusieurs des amorces ont une séquence nucléotidique complémentaire de l'un des brins du génome circulaire, et une ou plusieurs des amorces ont une séquence nucléotidique complémentaire de l'autre brin du génome circulaire, dans lequel l'ensemble des amorces ont une séquence nucléotidique complémentaire de l'un des brins du génome circulaire, ou l'ensemble des amorces ont une séquence nucléotidique complémentaire de l'autre brin du génome circulaire.

16. Procédé selon l'une quelconque des revendications 1 à 15, dans lequel le génome circulaire est un génome monobrin, dans lequel une ou plusieurs des amorces ont une séquence nucléotidique complémentaire du génome circulaire, et une ou plusieurs des amorces ont une séquence nucléotidique correspondant à une séquence du génome circulaire, dans lequel la réplication en cercle roulant résulte en la formation d'une séquence d'ADN répétée en tandem, dans lequel la ou les amorces ayant une séquence nucléotidique correspondant à une séquence du génome circulaire amorce(nt) une réplication par déplacement de brin de la séquence d'ADN répétée en tandem, dans lequel la réplication de la séquence d'ADN répétée en tandem résulte en la formation d'une séquence d'ADN secondaire répétée en tandem.

17. Procédé selon l'une quelconque des revendications 1 à 16, dans lequel les amorces comprennent chacune séparément des désoxyribonucléotides, des ribonucloétides, des nucléotides modifiés, des analogues de nucléotides, des nucléotides marqués, des analogues oligomères, ou une combinaison.

18. Procédé selon l'une quelconque des revendications 1 à 17, dans lequel l'échantillon d'acide nucléique génomique est un échantillon de sang, un échantillon d'urine, un échantillon de sperme, un échantillon de fluide lymphatique, un échantillon de fluide céphalorachidien, un échantillon de fluide amniotique, un échantillon de biopsie, un échantillon de biopsie obtenue par aspiration à l'aiguille, un échantillon de cellules cancéreuses, un échantillon de cellules tumorales, un échantillon de tissus, un échantillon de cellules, un échantillon de lysat de cellules, un échantillon brut de lysat de cellules, un échantillon médico-légal, un échantillon archéologique, un échantillon infectieux, un échantillon de cellules infectieuses nosocomiales, un échantillon de l'environnement, ou une combinaison de ceux-ci.


### SEQUENCE LISTING

<110> QIAGEN GMBH
   CHRISTIAN KORFHAGE
   DIRK LOFFERT
<120> ROLLING CIRCLE AMPLIFICATION OF CIRCULAR GENOMES
<130> N3039 EP BLN
<150> 60/862,678
   <151> 2006-10-24
<160> 20
<170> FastSEQ for Windows Version 4.0
<210> 1
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 1
   ccccattcca 10
<210> 2
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 2
   caattgagta 10
<210> 3
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 3
   aatccttcta 10
<210> 4
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 4
   cggtctgtta 10
<210> 5
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 5
   gccacaacta 10
<210> 6
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 6
   tatgagagta 10
<210> 7
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 7
   caaacatcta 10
<210> 8
   <211> 10
   <212> DNA
   <213> Artificial Sequence
<220>
   <223> Description of Artificial Sequence: Synthetically constructed Mitochondrial specific primer
<220>
   <221> modified_base
   <222> 8, 9
   <223> Exonuclease resistant Phosphorothioate Modification
<400> 8
   cgtggttgta 10
<210> 9
   <211> 14
   <212> DNA
   <213> Artificial Sequence
<220>
   <223> Description of Artificial Sequence: Synthetically constructed Mitochondrial specific primer
<220>
   <221> modified_base
   <222> 12, 13
   <223> Exonuclease resistant Phosphorothioate
<400> 9
   gcatccccat tcca 14
<210> 10
   <211> 14
   <212> DNA
   <213> Artificial Sequence
<220>
   <223> Description of Artificial Sequence: Synthetically constructed Mitochondrial specific primer
<220>
   <221> modified_base
   <222> 12, 13
   <223> Exonuclease resistant Phosphorothioate Modification
<400> 10
   ttcccaattg agta 14
<210> 11
   <211> 14
   <212> DNA
   <213> Artificial Sequence
<220>
   <223> Description of Artificial Sequence: Synthetically constructed Mitochondrial specific primer
<220>
   <221> modified_base
   <222> 12, 13
   <223> Exonuclease resistant Phosphorothioate Modification
<400> 11
   ccataatcct tcta 14
<210> 12
   <211> 14
   <212> DNA
   <213> Artificial Sequence
<220>
   <223> Description of Artificial Sequence: Synthetically constructed Mitochondrial speciific primer
<220>
   <221> modified_base
   <222> 12, 13
   <223> Exonuclease resistant Phosphorothioate Modification
<400> 12
   gttgcggtct gtta 14
<210> 13
   <211> 14
   <212> DNA
   <213> Artificial Sequence
<220>
   <223> Description of Artificial Sequence: Synthetically constructed Mitochondrial specific primer
<220>
   <221> modified_base
   <222> 12, 13
   <223> Exonuclease resistant Phosphorothioate Modification
<400> 13
   tattgccaca acta 14
<210> 14
   <211> 14
   <212> DNA
   <213> Artificial Sequence
<220>
   <223> Description of Artificial Sequence: Synthetically constructed Mitochondrial specific primer
<220>
   <221> modified_base
   <222> 12, 13
   <223> Exonuclease resistant Phosphorothioate Modification
<400> 14
   gggttatgag agta 14
<210> 15
   <211> 14
   <212> DNA
   <213> Artificial Sequence
<220>
   <223> Description of Artificial Sequence: Synthetically constructed Mitochondrial specific primer
<220>
   <221> modified_base
   <222> 12, 13
   <223> Exonuclease resistant Phosphorothioate Modification
<400> 15
   gacccaaaca tcta 14
<210> 16
   <211> 14
   <212> DNA
   <213> Artificial Sequence
<220>
   <223> Description of Artificial Sequence: Synthetically constructed Mitochondrial specific primer
<220>
   <221> modified_base
   <222> 12, 13
   <223> Exonuclease resistant Phosphorothioate Modification
<400> 16
   tggtcgtggt tgta 14
<210> 17
   <211> 18
   <212> DNA
   <213> Artificial Sequence
<220>
   <223> Description of Artificial Sequence: Synthetically constructed nuclear DNA specific primer
<400> 17
   tgctccctgt cccatctg 18
<210> 18
   <211> 24
   <212> DNA
   <213> Artificial Sequence
<220>
   <223> Description of Artificial Sequence: Synthetically constructed nuclear DNA specific primer
<400> 18
   agacagtatg cctttatttc accc 24
<210> 19
   <211> 20
   <212> DNA
   <213> Artificial Sequence
<220>
   <223> Description of Artificial Sequence: note = synthetic construct
<400> 19
   ctcttgctca gcctatatac 20
<210> 20
   <211> 21
   <212> DNA
   <213> Artificial Sequence
<220>
   <223> Description of Artificial Sequence: note = synthetic construct
<400> 20
   gtagaaaatg tagcccatta c 21